# EUROPÄISCHE PATENTSCHRIFT

(11) **EP 0 751 218 B1**
(45) Veröffentlichungstag und Bekanntmachung des Hinweises auf die Patenterteilung: **01.09.2004**
(21) Anmeldenummer: 96110396.7
(22) Anmeldetag: 27.06.1996
(51) Int. Cl.: C12N 15/01, C12N 15/74, C12N 15/61, C12P 19/24, C12N 1/21, C12N 15/54

(54) **Saccharose-Stoffwechsel-Mutanten**
Sucrose-metabolism mutants
Mutants du métabolisme de saccharose

(30) Priorität: 28.06.1995 DE 19523560
(43) Veröffentlichungstag der Anmeldung: 02.01.1997
(73) Patentinhaber: SÜDZUCKER AKTIENGESELLSCHAFT MANNHEIM/OCHSENFURT, 68165 Mannheim (DE)
(72) Erfinder: Mattes, Ralf, Prof. Dr., 70619 Stuttgart (DE); Klein, Kathrin, Dr. rer. nat., 70197 Stuttgart (DE); Stegmaier, Sabine, Dipl.-Biol., 70619 Stuttgart (DE)
(74) Vertreter: Weiss, Wolfgang, Dipl.-Chem. Dr.

(56) Entgegenhaltungen:
- EP-A- 0 483 755
- EP-A- 0 509 834
- SPRENGER G A ET AL: "Analysis of sucrose catabolism in Klebsiella pneumoniae and in Scr+ derivatives of Escherichia coli K12." JOURNAL OF GENERAL MICROBIOLOGY, (1988 JUN) 134 ( PT 6) 1635-1644, XP002100700
- BRÜCKNER R ET AL: "Characterization of a sucrase gene from Staphylococcus xylosus." JOURNAL OF BACTERIOLOGY, (1993 FEB) 175 (3) 851-857, XP002100701

## Beschreibung

Die vorliegende Erfindung betrifft ein verbessertes Verfahren zur Herstellung von nicht-kariogenen Zuckern, insbesondere Trehalulose oder/und Palatinose, unter Verwendung rekombinanter DNA-Technologie.

Die akariogenen Zuckerersatzstoffe Palatinose (Isomaltulose) und Trehalulose werden großtechnisch aus Saccharose durch eine enzymatische Umlagerung unter Verwendung von immobilisierten Bakterienzellen (z.B. der Spezies Protaminobacter rubrum, Erwinia rhapontici, Serratia plymuthica) hergestellt. Dabei wird die zwischen den beiden Monosaccharideinheiten des Disaccharids Saccharose bestehende α1 → β2 glykosidische Bindung zu einer α1 → 6 Bindung bei Palatinose bzw. einer α1 → α1 Bindung bei Trehalulose isomerisiert. Diese Umlagerung von Saccharose zu den beiden akariogenen Disacchariden erfolgt unter Katalyse des bakteriellen Enzyms Saccharose-Isomerase, auch Saccharose-Mutase genannt. Je nach verwendetem Organismus ergibt sich bei dieser Reaktion ein Produktgemisch, das neben den erwünschten akariogenen Disacchariden Palatinose und Trehalulose auch gewisse Anteile an unerwünschten Monosacchariden (Glucose oder/und Fructose) enthält. Diese Monosaccharidanteile sind ein erhebliches technisches Problem, da zu ihrer Abtrennung aufwendige Reinigungsprozeduren (meist fraktionierte Kristallisationen) erforderlich sind.

Bei der Produktion von akariogenen Disacchariden können Monosaccharide einerseits durch Abbau der gewünschten Endprodukte Palatinose oder/und Trehalulose und andererseits durch Abbau des Ausgangsprodukts Saccharose erzeugt werden. In PCT/EP/95/00165 werden Zellen vorgeschlagen, die einen reduzierten Palatinose oder/und Trehalulose-Stoffwechsel aufweisen, d.h. diese Disaccharide nur in geringem Umfang metabolisch verwerten können. Zellen, die einen verringerten Saccharose-Stoffwechsel aufweisen, werden in PCT/EP/95/00165 nicht offenbart.

Eine der vorliegenden Erfindung zugrundeliegende Aufgabe bestand somit darin, den Abbau von Saccharose zu Monosacchariden bei der Herstellung von Trehalulose oder/und Palatinose möglichst weitgehend zu unterdrücken. Eine andere, der vorliegenden Erfindung zugrundeliegende Aufgabe bestand in der Bereitstellung von Organismen, die Palatinose oder/und Trehalulose in höherer Ausbeute als bekannte Organismen erzeugen.

Zur Lösung dieser Aufgaben werden im Saccharose-Stoffwechsel defiziente Zellen, ein Verfahren zu deren Herstellung, sowie ein verbessertes Verfahren zur Herstellung von nicht-kariogenen Zuckern, insbesondere von Palatinose oder/und Trehalulose, bereitgestellt.

Ein Gegenstand der Erfindung ist eine Zelle, die mindestens eine für ein Protein mit einer Saccharose-Isomerase-Aktivität codierende DNA-Sequenz enthält und einen reduzierten Saccharose-Stoffwechsel aufweist.

Im Zusammenhang mit der vorliegenden Erfindung soll der Begriff "Protein mit einer Saccharose-Isomerase-Aktivität" solche Proteine umfassen, die zur Isomerisierung von Saccharose zu anderen Disacchariden in der Lage sind, wobei die α1 → β2 glykosidische Bindung zwischen Glucose und Fructose in der Saccharose in eine andere glykosidische Bindung zwischen zwei Monosaccharideinheiten überführt wird, insbesondere in eine α1 → 6 Bindung oder/und eine α1 → α1 Bindung. Besonders bevorzugt betrifft der Begriff "Protein mit einer Saccharose-IsomeraseAktivität" daher ein Protein, das zur Isomerisierung von Saccharose zu Palatinose oder/und Trehalulose in der Lage ist. Dabei beträgt der Anteil von Palatinose und Trehalulose an den gesamten Disacchariden, die durch Isomerisierung von Saccharose gebildet werden, vorzugsweise ≥ 2 %, besonders bevorzugt ≥ 20 % und am meisten bevorzugt ≥ 50 %.

Beispiele für Zellen, die für ein Protein mit Saccharose-Isomerase-Aktivität codierende Nukleotidsequenzen enthalten, sind insbesondere Mikroorganismen der Gattungen Protaminobacter, Erwinia, Serratia, Leuconostoc, Pseudomonas, Agrobacterium, Klebsiella und Enterobacter. Spezifische Beispiele für solche Mikroorganismen sind Protaminobacter rubrum (CBS 547,77), Erwinia rhapontici (NCPPB 1578), Serratia plymuthica (ATCC 15928), Serratia marcescens (NCIB 8285), Leuconostoc mesenteroides NRRL B-521f (ATCC 10830a), Pseudomonas mesoacidophila MX-45 (FERM 11808 bzw. FERM BP 3619), Agrobacterium radiobacter MX-232 (FERM 12397 bzw. FERM BP 3620), Klebsiella subspezies und Enterobacter spezies.

Spezifische Beispiele für Proteine mit Saccharose-Isomerase-Aktivität und dafür codierende Nukleinsäuren aus P.rubrum, E.rhapontici, Enterobacter spec. SZ 62 und P. mesoacidophila sind in PCT/EP 95/00165 beschrieben. Auf die Offenbarung dieser Patentanmeldung wird hiermit Bezug genommen.

Der Begriff "reduzierter Saccharose-Stoffwechsel" bedeutet im Sinne der vorliegenden Erfindung, daß eine Zelle in geringerem Umfang als eine entsprechende Wildtypzelle Saccharose metabolisch durch Abbau zu Monosacchariden verwerten kann. Dies bedeutet, daß in erfindungsgemäßen Zellen mindestens ein Stoffwechselweg der einen Abbau von Saccharose zu unerwünschten Nebenprodukten, insbesondere Glucose oder Fructose bewirkt, teilweise oder vollständig blockiert ist. In erfindungsgemäßen Zellen erfolgt die metabolische Verwertung von Saccharose daher verstärkt durch Isomerisierung der Saccharose zu akariogenen Disacchariden, insbesondere Palatinose und Trehalulose. Eine erfindungsgemäße Zelle kann daher höhere Anteile der nicht-kariogenen Disaccharide Trehalulose oder/und Palatinose und verringerte Mengen an den störenden Nebenprodukten Glucose bzw. Fructose als eine Wildtypzelle erzeugen. Die Reduzierung des Saccharose-Stoffwechsels kann beispielsweise durch teilweise oder vollständige Hemmung der Expression von mindestens einem Gen erfolgen, das für ein Saccharose-Stoffwechsel-Enzym codiert. Wenn die Wildtypzelle, die als Ausgangsmaterial zur Herstellung von Saccharose-Stoffwechsel-Mutanten verwendet wird, über mehr als einen Stoffwechselweg zur Verwertung von Saccharose verfügt, ist es bevorzugt, möglichst alle Stoffwechselwege zu hemmen oder zu blockieren.

In der vorliegenden Anmeldung ist die Isolierung von Genabschnitten beschrieben, welche für Enzyme des Saccharose-Stoffwechsels aus P.rubrum und Enterobacter spec. SZ 62 codieren. Von diesen Genabschnitten wurden auch Teilsequenzen bestimmt, die teilweise homolog zu bekannten Genen für den Saccharose-Stoffwechsel aus Salmonella oder Klebsiella sind. Diese isolierten Genabschnitte verleihen damit transformierten E.coli Zellen die Fähigkeit zur Verstoffwechselung von Saccharose.

Zur Herstellung von erfindungsgemäßen Zellen mit verringertem Saccharose-Stoffwechsel werden aus den isolierten Genabschnitten durch Verdau mit Restriktionsendonukleasen Teile deletiert. Auf diese Weise können DNA-Sequenzen identifiziert werden, deren vollständige oder teilweise Entfernung zur Inaktivierung des Saccharose-Stoffwechsels in E.coli führt. Diese Deletionen lassen sich beispielsweise über entsprechende bekannte Verfahren in andere Mikroorganismen zurückkreuzen und über homologe Rekombination im Chromosom etablieren. Dadurch entstehen erfindungsgemäße Deletionsmutanten der Ausgangsstämme, die die Fähigkeit zur Saccharose-Verstoffwechselung dauerhaft verloren haben. Es werden Beispiele für derartige Deletionsmutanten von Protaminobacter rubrum und Enterobacter SZ 62 beschrieben.

Bei einer Untersuchung der erfindungsgemäß hergestellten Deletionsmutanten wurde festgestellt, daß im Vergleich zum Wildtyp mit intaktem Saccharosestoffwechsel wesentlich geringere Monosaccharid-Konzentrationen erhalten werden. Dieser Erfolg wird sowohl bei Ansätzen im Reagenzglas als auch bei immobilisierten Zellen im Labormaßstab erreicht.

Weiterhin wurde überraschenderweise gefunden, daß eine Saccharose-Deletionsmutante von Protaminobacter rubrum die Saccharose-Isomerase konstitutiv exprimieren kann. Daher ist eine Produktion von akariogenen Zuckern sogar ohne Zusatz von Saccharose im Medium möglich.

Es wurde eine Reihe von Genen aus Protaminobacter rubrum und Enterobacter spezies SZ 62 identifiziert, die für Saccharose-Stoffwechselenzyme codieren. Diese Gene sind auf den Plasmiden pKAT 101, pKAT 102, pKAT 103 und pSST 3001 lokalisiert. Diese Plasmide wurden bei der Deutschen Sammlung von Mikroorganismen und Zellkulturen GmbH (DSM), Mascheroder Weg 1b, D-38124 Braunschweig unter den Hinterlegungsnummern 10030 (pKAT 101), 10031 (pKAT 102), 10032 (pKAT 103) und 10033 (pSST 3001) nach den Vorschriften des Budapester Vertrages hinterlegt.

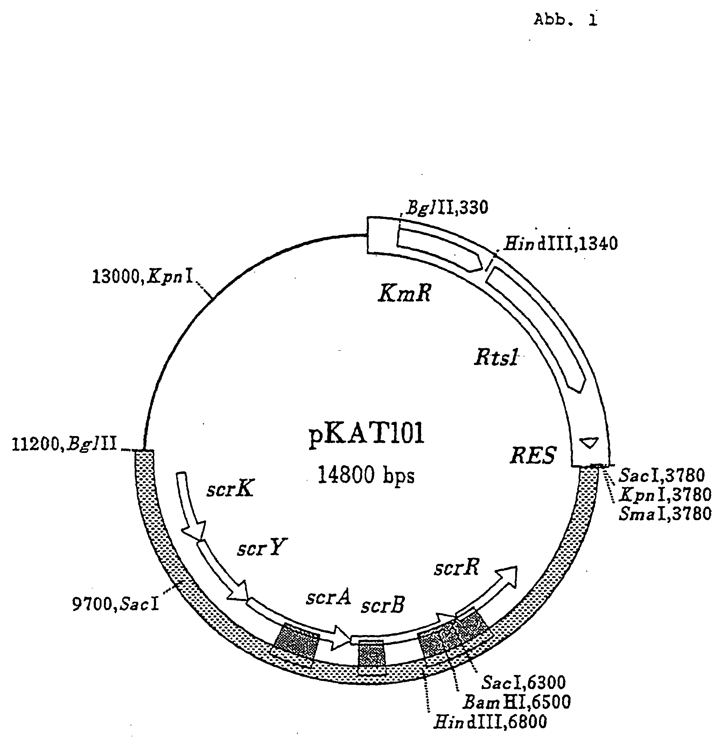

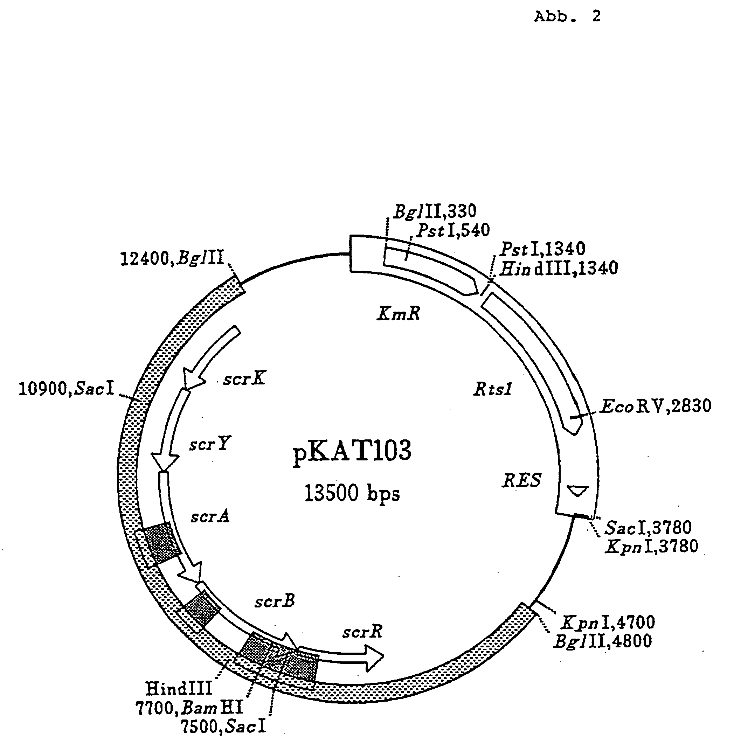

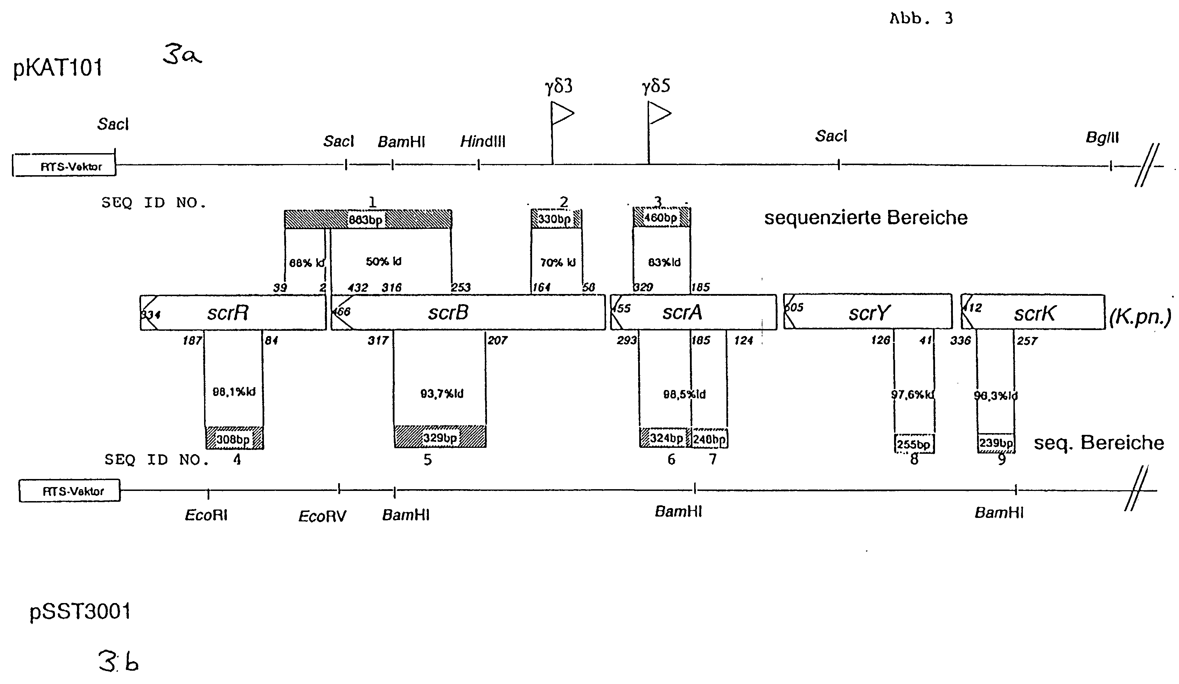

Der Gegenstand einer ersten Ausführungsform der vorliegenden Erfindung ist somit eine Zelle, bei der die Reduzierung des Saccharose-Stoffwechsels durch teilweise oder vollständige Hemmung der Expression von DNA-Sequenzen auf den Plasmiden pKAT 101 (DSM 10030) und pKAT 103 (DSM 10032) oder dazu homologen DNA-Sequenzen erfolgt. Die Homologie zu den Sequenzen auf pKAT 101 und pKAT 103 ist vorzugsweise mindestens 70 %, besonders bevorzugt mindestens 80 % und am meisten bevorzugt mindestens 90 % auf Nucleotidebene. Die DNA-Sequenzen auf pKAT 101 und pKAT 103 wurden aus P.rubrum isoliert und codieren für Enzyme, die an der Verstoffwechslung von Saccharose beteiligt sind. Durch Deletion von einem oder mehreren Genabschnitten kann die P.rubrum-Zelle ihre Fähigkeit zur Verwertung von Saccharose über den betroffenden Stoffwechselweg verlieren. Teilabschnitte der DNA-Sequenzen aus pKAT 101 sind in den beiliegenden Sequenzprotokollen SEQ ID NO. 1-3 gezeigt. Diese Sequenzen sind auf Ebene der translatierten Bereiche zu 68 - 83 % homolog zu Abschnitten der Gene für scrR, scrB und scrA des Bakteriums Klebsiella pneumoniae (Jahreis und Lengeler, Mol. Microbiol, 9 (1993), 195 - 209). In Abb. 1 und 2 sind die Plasmidkarten von pKAT 101 und pKAT 103 mit Angabe der sequenzierten Teilabschnitte dargestellt. Abb. 3 a zeigt einen Vergleich der sequenzierten Abschnitte mit den dazu homologen Regionen aus K.pneumoniae.

Neben den in den Sequenzprotokollen dargestellten Nukleotidsequenzen umfaßt die vorliegende Erfindung auch noch DNA-Sequenzen, die mit einer dieser Sequenzen hybridisieren. Der Begriff "Hybridisierung" gemäß vorliegender Erfindung wird, wie bei Sambrook et al (Molecular Cloning. A Laboratory Manual, ColdSpring Harbor Laboratory Press (1989), 1.101-1.104) verwendet. Gemäß vorliegender Erfindung spricht man von einer Hybridisierung, wenn nach Waschen für eine Stunde mit 1x SSC und 0,1 % SDS bei 55°C, vorzugsweise bei 62°C und besonders bevorzugt bei 68°C, insbesondere für 1 Stunde in 0,2 X SSC und 0,1 % SDS bei 55°C, vorzugsweise bei 62°C und besonders bevorzugt bei 68°C noch ein positives Hybridisierungssignal beobachtet wird. Eine unter derartigen Waschbedingungen mit einer der in den Sequenzprotokollen angegebenen Nukleotidsequenzen hybridisierende Nukleotidsequenz ist eine erfindungsgemäße Nukleotidsequenz.

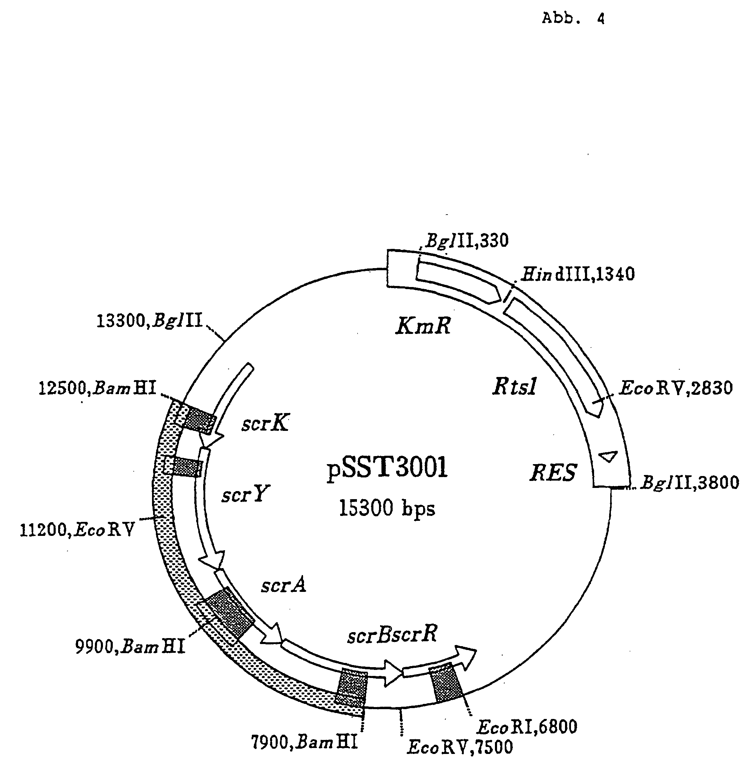

Der Gegenstand einer zweiten Ausführungsform der vorliegenden Erfindung ist eine Zelle, in der die Reduzierung des Saccharose-Stoffwechsels durch teilweise oder vollständige Hemmung der Expression von DNA-Sequenzen auf dem Plasmid pSST 3001 (DSM 10033) oder dazu homologen Sequenzen erfolgt. Die DNA-Sequenzen auf pSST 3001 wurden aus Enterobacter spec. SZ62 isoliert und codieren für Enzyme, die an der Verstoffwechselung von Saccharose beteiligt sind. Durch Deletion von einem oder mehreren Genabschnitten kann die Enterobacter-Zelle ihre Fähigkeit zur Verwertung von Saccharose über den betreffenden Stoffwechselweg verlieren. Vorzugsweise werden die DNA-Sequenzen ausgewählt aus den in den Sequenzprotokollen SEQ ID NO. 4-9 gezeigten Nukleotidsequenzen oder damit hybridisierenden Nukleotidsequenzen. Die ermittelten Sequenzen haben eine hohe Homologie zu Bereichen des scr-Operons von K. pneumoniae (Jahreis und Lengeler, Supra). Die sequenzierten Teilbereiche sind auf Ebene der translatierten Sequenz zu 93-99 % homolog zu den Abschnitten für scrK, scrY, scrB, scrA und scrR aus K. pneumoniae. In Abb. 4 ist die Plasmidkarte von pSST 3001 mit Angabe der sequenzierten Teilabschnitte gezeigt. Abb. 3b zeigt einen Vergleich dieser Abschnitte mit den dazu homologen Regionen aus K.pneumoniae.

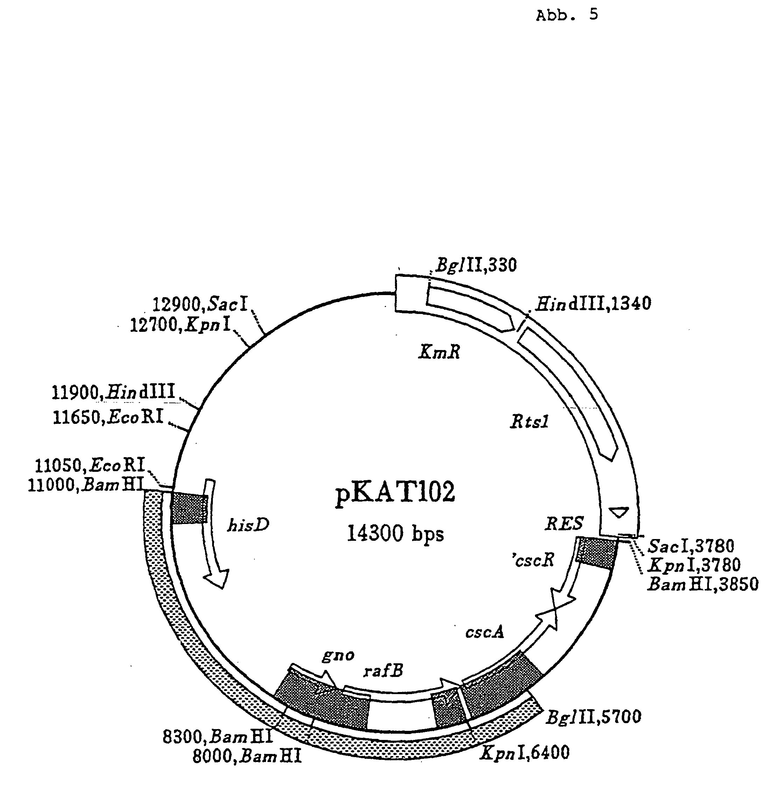

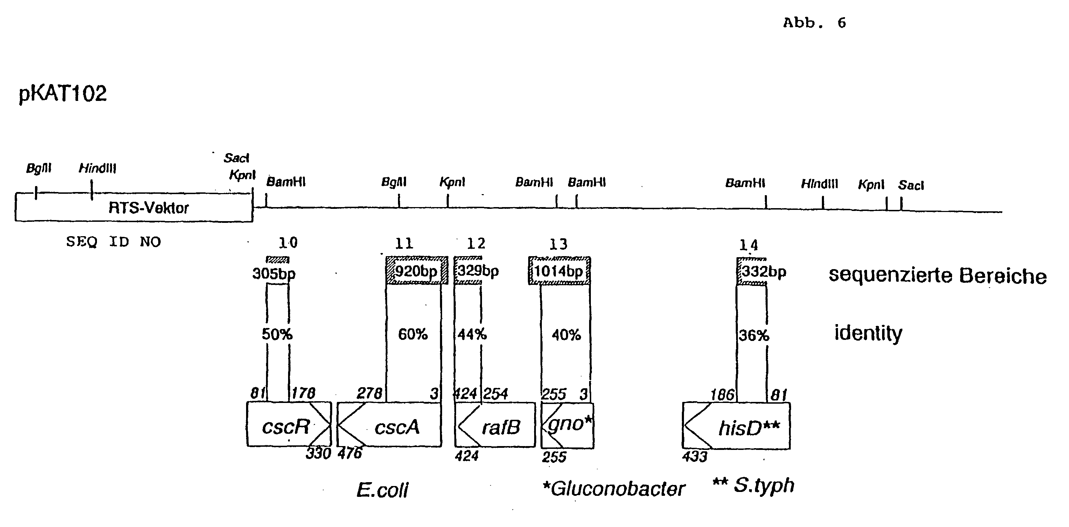

In einer dritten Ausführungsform der vorliegenden Erfindung erfolgt die Reduzierung des Saccharose-Stoffwechsels durch teilweise oder vollständige Hemmung der Expression von DNA-Sequenzen auf dem Plasmid pKAT 102 (DSM 10031) oder dazu homologen DNA-Sequenzen. Die DNA-Sequenzen auf pKAT 102 stammen aus P.rubrum. Vorzugsweise werden die DNA-Sequenzen ausgewählt aus den in den Sequenzprotokollen SEQ ID NO. 10 - 14 gezeigten Nukleotidsequenzen oder damit hybridisierenden Nukleotidsequenzen. Die auf dem Plasmid pKAT 102 enthaltenen DNA-Sequenzen sind nicht identisch oder nahe verwandt mit denen in der Plasmide pKAT 101 und pSST 3001. Die ermittelten Sequenzen sind homolog zu Bereichen des csc-Operons aus E.coli bzw. zu Sequenzen aus Gluconobacter oder Salmonella typhimurium. In Abb. 5 ist die Plasmidkarte von pKAT 102 mit Angabe der sequenzierten Teilabschnitte dargestellt. Abb. 6 zeigt einen Vergleich dieser Abschnitte mit bekannten, dazu homologen bakteriellen Regionen.

Die erfindungsgemäße Zelle mit reduziertem Saccharose-Stoffwechsel kann die für ein Protein mit einer Saccharose-Isomerase-Aktivität codierende DNA-Sequenz als natürlichen Bestandteil ihres Chromosoms enthalten. Andererseits kann die Zelle auch mit einem Saccharose-Isomerase-Gen transformiert sein. Die Sequenzen von Saccharose-Isomerase-Genen aus Protaminobacter rubrum, Erwinia rhapontici, Enterobacter species SZ 62 und Pseudomonas mesoacidophila MX-45 sind in PCT/EP 95/00165 beschrieben. Diese Gene können in fremde prokaryontische oder eukaryontische Zellen eingeführt werden, wobei Zellen entstehen, die mit einem Saccharose-Isomerasegen transformiert sind.

In einer Ausführungsform ist diese Zelle eine prokaryontische Zelle, vorzugsweise eine gram-negative prokaryontische Zelle, besonders bevorzugt eine Enterobakterienzelle. Dabei kann einerseits eine Zelle verwendet werden, die kein eigenes Saccharose-Isomerasegen enthält, wie z.B. E.coli, andererseits können aber auch Zellen verwendet werden, die bereits ein solches Gen auf ihrem Chromosom enthalten, z.B. die oben als Quelle für Saccharose-Isomerasegene genannten Mikroorganismen. Bevorzugte Beispiele für geeignete prokaryontische Zellen sind E.coli-, Protaminobacter rubrum-, Erwinia rhapontici-, Enterobacter spec.- oder Pseudomonas mesoacidophila-Zellen. Die Transformation prokaryontischer Zellen mit exogenen Nukleinsäuresequenzen ist einem Fachmann auf dem Gebiet der Molekularbiologie geläufig (vgl. z.B. Sambrook et al., supra, Kapitel 1 - 4).

In einer weiteren Ausführungsform der vorliegenden Erfindung kann die erfindungsgemäße Zelle jedoch auch eine eukaryontische Zelle sein, wie etwa eine Pilzzelle (z.B. Hefe), eine tierische oder eine pflanzliche Zelle. Verfahren zur Transformation bzw. Transfektion eukaryontischer Zellen mit exogenen Nukleinsäuresequenzen sind dem Fachmann auf dem Gebiet der Molekularbiologie ebenfalls geläufig und brauchen hier nicht näher erläutert zu werden (vgl. z.B. Sambrook et al., Kapitel 16).

Überraschenderweise konnten auch Saccharose-Stoffwechsel-Mutanten identifiziert werden, die das Protein mit Saccharose-Isomerase-Aktivität konstitutiv, d.h. ohne Zusatz von Saccharose zum Medium, produzieren. Vorzugsweise ist bei diesen Zellen der Repressor des scr-Operons teilweise oder vollständig deletiert.

Spezifische Beispiele für erfindungsgemäße Saccharose-Stoffwechsel-Mutanten sind die Protamincbacter rubrum-Stämme iiG Pr 20191 und iiG Pr 20710. Ein weiteres Beispiel ist der Enterobacter spec. SZ 62-Stamm iiG ES 2111, der bei DSM unter der Hinterlegungsnummer DSM 10025 hinterlegt wurde. Die P. rubrum-Mutantenstämme sind in der Lage, 0,5 bis 1,5 U/mg Saccharose-Isomerase ohne Zusatz von Saccharose zum Medium zu produzieren. Im Vergleich dazu produziert der P.rubrum Wildtypstamm unter gleichen Bedingungen nur ≤ 0,1 U/mg Isomerase. Der Enterobacter-Mutantenstamm weist die überraschende Eigenschaft auf, daß er die Saccharose-Isomerase nicht unter Zusatz von Saccharose zum Medium produziert. Hingegen führt ein Zusatz von Palatinose zum Medium zu einer im Vergleich zum Wildtyp erhöhten Produktion des Enzyms. Bei dieser Mutante scheint das Aufnahmesystem für Saccharose defekt zu sein, d.h. die Zelle ist nicht mehr in der Lage, Saccharose aufzunehmen und sie oder ein Umwandlungsprodukt als Induktor zu verwenden. Für Palatinose besteht ein selektives Aufnahmesystem, als Induktor scheint entweder Palatinose selbst oder ein Stoffwechselfolgeprodukt davon zu fungieren.

Ein bevorzugter Weg zur Herstellung von erfindungsgemäßen Saccharose-Defekt-Mutanten besteht darin, in einem bestimmten Organismus Gene, die für Saccharose-Stoffwechsel-Enzyme codieren, zu identifizieren und teilweise oder vollständig zu deletieren. In vielen Organismen gelingt diese Deletion durch Einführung eines zur homologen chromosomalen Rekombination geeigneten Vektors, der ein mutiertes Saccharose-Stoffwechselgen trägt, und Selektionen von Organismen, in denen eine derartige Rekombination stattgefunden hat. Das Prinzip der Selektion durch genetische Rekombination wird bei E. L. Winnacker, Gene und Klone, Eine Einführung in die Gentechnologie, VCH-Verlagsgesellschaft Weinheim, BRD, S. 320 ff. erläutert.

Die Identifizierung von Genen, die für Saccharose-Stoffwechsel-Enzyme codieren, kann einerseits durch Homologievergleiche mit den in der vorliegenden Anmeldung offenbarten Nukleotidsequenzen und andererseits durch ein im folgenden beschriebenes Selektionsverfahren erfolgen. Bei diesem Selektionsverfahren wird aus einem Spenderorganismus, der für Saccharose-Stoffwechselenzyme codierende Gene enthält, die chromosomale DNA isoliert, in Fragmente der gewünschten Größe gespalten und in einen Wirtsorganismus eingeführt, welcher selbst nicht in der Lage ist, Saccharose zu verwerten. Ein Beispiel für einen geeigneten Wirtsorganismus ist E.coli. Die Identifizierung von Kolonien, welche für Saccharose-Stoffwechselenzyme codierende Gene enthalten, kann durch Kultivierung auf McConkey-Saccharose-Medium und Musterung auf rot gefärbte Kolonien erfolgen. Unter den auf diese Weise identifizierten rot gefärbten Kolonien werden wiederum solche ausgewählt, die zur Spaltung von Saccharose in Glucose und Fructose fähig sind.

Andererseits können Saccharose-Defekt-Mutanten auch durch unspezifische Mutagenese aus geeigneten Ausgangsorganismen und Selektion der Saccharose-Defekt-Mutanten erhalten werden. Bei Verwendung von Ausgangsorganismen mit Defekten im Palatinose-Stoffwechsel kann eine Selektion auf Saccharose-Defekt-Mutanten durch Verwendung von McConkey-Saccharose-Medien oder Minimalsalz-Medien mit Saccharose als einziger C-Quelle erfolgen. Die Mutanten, die auf McConkey-Saccharose-Medium weiß sind oder auf Minimalsalz-Medien mit Glucose als einziger C-Quelle, aber nicht auf entsprechenden Medien mit Saccharose als einziger C-Quelle wachsen, werden als Saccharose-Defektmutanten identifiziert. Bei Verwendung anderer Ausgangsorganismen ist die Verwendung eines zusätzlichen Selektionsmarkers, z.B. eines Antibiotikum-Resistenzgens zweckmäßig.

Der Gegenstand der vorliegenden Erfindung ist somit auch ein Verfahren zur Herstellung von Zellen, die mindestens eine für ein Protein mit einer Saccharose-Isomerase-Aktivität codierende DNA-Sequenz enthalten und einen reduzierten Saccharose-Stoffwechsel aufweisen, wobei man durch Mutagenese der Zelle mindestens ein Gen inaktiviert, das für ein Saccharose-Stoffwechselenzym codiert. Diese Inaktivierung kann beispielsweise durch teilweise oder vollständige Deletion der Protein-codierenden Sequenz oder durch teilweise oder vollständige Deletion einer regulatorischen Sequenz erfolgen. Vorzugsweise erfolgt die Mutagenese durch Einführung eines zur homologen chromosomalen Rekombination geeigneten Vektors, der eine mutierte, für den Saccharose-Stoffwechsel essentielle DNA-Sequenz trägt, und Selektion von Zellen, in denen eine derartige Rekombination stattgefunden hat.

Ein weiterer Aspekt der vorliegenden Erfindung sind Zellen, die neben einem reduzierten Saccharose-Stoffwechsel auch einen reduzierten Palatinose- oder/und Trehalulose-Stoffwechsel aufweisen. Derartige Zellen erzeugen noch höhere Anteile der nicht- kariogenen-Disaccharide Trehalulose oder/und Palatinose sowie verringerte Mengen an den störenden Produkten Glucose bzw. Fructose.

Der Begriff "reduzierter Palatinose- oder/und Trehalulosestoffwechsel" bedeutet im Sinne der vorliegenden Erfindung, daß eine ganze Zelle des betreffenden Organismus bei der Verwertung von Saccharose als C-Quelle akariogene Disaccharide erzeugt und diese aber nur in geringem Umfang metabolisch verwerten kann, z.B. indem sie zu Monosacchariden abgebaut werden. Vorzugsweise erzeugt der Organismus weniger als 2,5 %, besonders bevorzugt weniger als 2 %, am meisten bevorzugt weniger als 1 % Glucose plus Fructose auf Basis der Summe von akariogenen Disacchariden und Monosaccharidabbauprodukten bei einer Temperatur von 15 - 65°C, insbesondere von 25 - 55°C.

In einer Ausführungsform der vorliegenden Erfindung kann die Reduzierung des Palatinose- oder/und Trehalulosestoffwechsels durch teilweise oder vollständige Hemmung der Expression von Palatinasegenen erfolgen, die für den intrazellulären Abbau von Palatinose oder/und Trehalulose verantwortlich sind. Diese Hemmung der Genexpression kann beispielsweise durch zielgerichtete Mutation oder/und Deletion der betreffenden Gene erfolgen. Spezifische Beispiele für Palatinasegene aus Protaminobacter rubrum und Pseudomonas mesoacidophila sind in PCT/EP 95/00165 angegeben. Auf diese Offenbarung wird hiermit Bezug genommen.

Die Organismen mit reduziertem Palatinose- oder/ und Trehalulose-Stoffwechsel können weiterhin durch unspezifische Mutagenese aus geeigneten Ausgangsorganismen und Selektion der entsprechenden Defektmutanten erhalten werden. Ein Beispiel für eine derartige Palatinase-Defektmutante ist der Protaminobacter rubrum-Stamm SZZ 13, der am 29. März 1994 bei DSM unter der Hinterlegungsnummer DSM 9121 gemäß den Vorschriften des Budapester Vertrags hinterlegt wurde. Dieser Mikroorganismus wurde durch unspezifische Mutagenese von P. rubrum-Wildtypzellen mit N-Methyl-N'-nitro-N-nitrosoguanidin hergestellt und zeichnet sich dadurch aus, daß er die nicht-kariogenen Zucker Trehalulose und Palatinose nicht mehr in Glucose und Fructose spalten kann. Die Selektion solcher Mutanten kann z.B. durch Verwendung von MacConkey--Palatinose-Medien oder Minimalsalzmedien mit Palatinose oder Glucose als einziger C-Quelle erfolgen. Die Mutanten, die auf MacConkey-Palatinose-Medium (MacConkey-Agar-Base von Difco Laboratories, Detroit, Michigan, USA (40 g/l) und 10 g/l Palatinose) weiß sind oder auf Minimalsalzmedien mit Glucose als einziger C-Quelle, aber nicht auf entsprechenden Medien mit Palatinose als einziger C-Quelle wachsen, werden als Palatinase-Defekt-Mutanten identifiziert.

Ein derartiger Organismus mit reduziertem Palatinose-oder/und Trehalulose-Stoffwechsel kann als Ausgangsorganismus für eine unspezifische Mutagenese verwendet werden, um eine Saccharose-Defekt-Mutante zu erhalten.

Ein weiterer Gegenstand der vorliegenden Erfindung ist ein Verfahren zur Herstellung von nicht-kariogenen Zuckern, insbesondere Trehalulose oder/und Palatinose, welches dadurch gekennzeichnet ist, daß man zur Produktion der Zucker
(a) eine Zelle, die mindestens eine für ein Protein mit einer Saccharose-Isomerase-Aktivität codierende DNA-Sequenz enthält und einen reduzierten Saccharose-Stoffwechsel aufweist, oder/und
(b) einen Extrakt aus einer derartigen Zelle verwendet.

Die Durchführung des Verfahrens erfolgt im allgemeinen dadurch, daß man den Organismus bzw. den Extrakt in einem geeigneten Medium unter solchen Bedingungen kultiviert, bei denen Saccharose durch die Saccharose-Isomerase mindestens teilweise in akariogene Disaccharide umgewandelt wird. Anschließend werden die akariogenen Disaccharide aus dem Medium oder dem Organismus gewonnen und auf bekannte Weise aufgereinigt.

In einer bevorzugten Ausführungsform dieses Verfahrens wird der Organismus oder der Extrakt in immobilisierter Form verwendet. Die Immobilisierung von Proteinen (in reiner Form oder in Extrakten) erfolgt vorzugsweise über Kopplung von reaktiven Seitengruppen (z.B. NH₂-Gruppen) an einen geeigneten Träger. Die Immobilisierung von Zellen erfolgt z.B. in einer Natriumalginat/Calciumchlorid-Lösung. Ein Überblick über geeignete Methoden zur Immobilisierung von Zellen und Proteinen wird z.B. bei I. Chibata (Immobilized Enzymes, John Wiley and Sons, New York, London, 1978) gegeben.

Bei Verwendung einer mit dem Saccharose-Isomerase-Gen transformierten Zelle kann die Produktionsrate von akariogenen Zukkern gegenüber bekannten Organismen durch Erhöhung der Anzahl von Genkopien in der Zelle oder/und durch Erhöhung der Expressionsrate bei einer Kombination mit starken Promotoren gesteigert werden. Weiterhin kann durch Transformation einer Zelle, die nicht oder nur in beschränktem Ausmaß zur Verwertung von akariogenen Zuckern in der Lage ist, mit dem Saccharose-Isomerase-Gen eine transformierte Zelle erzeugt werden, mit deren Hilfe akariogene Zucker, insbesondere Palatinose oder/und Trehalulose, ohne oder mit weniger Nebenprodukten gewonnen werden können.

Bei Verwendung eines Mikroorganismus, der bereits ein funktionelles Saccharose-Isomerase-Gen enthält, ist die Transformation mit einem exogenen Saccharose-Isomerase-Gen nicht essentiell, kann aber zur Ausbeuteverbesserung durchgeführt werden.

Die in der vorliegenden Anmeldung offenbarten Nucleinsäuren, die für Enzyme des Saccharose-Stoffwechsels codieren, oder Teilfragmente davon können zur Herstellung von Mutagenese-Vektoren eingesetzt werden. Vorzugsweise werden diese Nucleinsäuren aus einem oder mehreren Genen von bakteriellen scr- oder/und csc-Operons ausgewählt, z.B. aus den Genen scrK, scrY, scrA, scrB, scrR, hisD, gno, rafB, cscA und cscR, oder Fragmenten davon. Besonders bevorzugt verwendet man eine Nucleinsäure, die (a) eine oder mehrere der in SEQ ID NO. 1-14 dargestellten Nucleotidsequenzen, (b) eine mit den Sequenzen aus (a) hybridisierende Sequenz oder/und (c) ein Fragment einer der Sequenzen aus (a) oder/und (b) umfaßt, wobei die Länge des Fragments vorzugsweise mindestens 100 Nucleotide beträgt.

Weiterhin wird die Erfindung durch folgende Sequenzprotokolle und Figuren beschrieben:

| | |
|---|---|
| SEQ ID NO. 1 - 3 | zeigen Teilsequenzen aus der Insertion des Plasmids pKAT 101, die für Saccharose-Stoffwechselenzyme aus P.rubrum codierende Abschnitte enthalten, |
| | |
| SEQ ID NO 4 - 9 | zeigen Teilsequenzen aus der Insertion des Plasmids pSST 3001, die für Saccharose-Stoffwechselenzyme aus Enterobacter species SZ 62 codierende Bereiche enthalten, |
| | |
| SEQ ID NO. 10 - 14 | zeigen Teilsequenzen der Insertionen aus pKAT 102, die für Saccharose-Stoffwechselenzyme aus P.rubrum codierende Bereiche enthalten, |
| | |
| Abb. 1 und 2 | zeigen die Plasmidkarten von pKAT 101 und pKAT 103 mit Angabe der sequenzierten Teilabschnitte, |
| | |
| Abb. 3 | zeigt einen Vergleich der sequenzierten Teilabschnitte aus pKAT 101 und pSST 3001 mit den dazu homologen Regionen aus K.pneumoniae, |
| | |
| Abb. 4 | zeigt die Plasmidkarte von pSST 3001 mit Angabe der sequenzierten Teilabschnitte, |
| | |
| Abb. 5 | zeigt die Plasmidkarte von pKAT 102 mit Angabe der sequenzierten Teilabschnitte und |
| | |
| Abb. 6 | zeigt einen Vergleich der sequenzierten Teilabschnitte aus pKAT 102 mit den dazu homologen Regionen aus E.coli, Gluconobacter und Salmonella typhimurium. |

### BEISPIEL 1

### Isolierung und Charakterisierung von scr-Genen für Enzyme des Saccharose-Stoffwechsels aus P.rubrum

Es wurde eine Genbank aus Protaminobacter rubrum Gesamt-DNA unter Verwendung von Sau3AI Partialverdaus des Genoms, Gewinnung von Fragmentscharen der Größe von ca 10kbp durch Elution der per Gelelektrophorese aufgetrennten Fragmente und Ligation in ein mit BamHI geöffnetes Derivat des λ EMBL4 Vektors, λRESII (J. Altenbuchner, Gene **123,** 63-68, 1993) hergestellt. Dann erfolgte eine Transfektion und Umwandlung der Phagen in Plasmide gemäß obigem Zitat. Die Plasmide wurden durch Transformation in E.coli Zellen eingeführt. Anschließend erfolgte ein Screening auf Rotfärbung der Kanamycin-resistenten (25 µg/ml) Zellen auf McConkey-Saccharose-Medium. Unter den gesuchten, rot gefärbten Kolonien werden solche ausgewählt, deren Rohextrakte zugesetzte Saccharose in Glucose und Fructose umsetzen. Dieser Nachweis erfolgt z.B. über die Kopplung der Reaktion mit GlucoseOxidase und Nachweis der H₂O₂-Bildung, alternativ über HPLC-Messung. Die aus diesen gewünschten Klonen isolierten Plasmide verleihen E.coli Wildtyp-Stämmen die Fähigkeit zum Wachstum auf Minimalsalzmedium mit Saccharose als einziger C-Quelle. Auf diese Weise wurden die Plasmide pKAT101 und pKAT 103 erhalten.

Die Abschnitte dieser Plasmide, die die gesuchten Gene enthalten, wurden durch Mutagenese mit einem γδ-Insertionselement (M. Strathmann et al, Proc. Natl. Acad. Sci. USA 88 (1991), 1247-1250) physikalisch kartiert. Plasmid-Mutanten, die den Zellen keine Saccharose-Verstoffwechselung mehr ermöglichen, enthalten diese über Restriktionsverdau nachweisbaren Insertionselemente in bestimmten Abschnitten der P.rubrum DNA. Schnittstellen für das Restriktionsenzym SacI flankieren diese Region.

Teilfragmente aus dieser Region von pKAT 101 wurden in M13-oder pUC-Vektoren subkloniert und die Sequenz der klonierten Abschnitte nach der Sanger-Methode bestimmt. Ein Vergleich der gewonnenen Sequenzdaten mit denen in GenBank zeigt auf, daß die ermittelten Teilsequenzen hohe Homologie zu Bereichen des scr-Regulons von Klebsiella pneumoniae (Jahreis, K. & J.W. Lengeler, Molecular Microbiology **9** 195-209, 1993) aufweisen. Die Teilbereiche sind auf Ebene der translatierten Sequenz zu 68-83 % homolog zu Teilen der Gene für scrR, scrB und scrA des beschriebenen Organismus. Die entsprechenden Sequenzabschnitte tragen die Bezeichnung SEQ ID NO. 1 (scrR-scrB), SEQ ID NO. 2 (scrB) und SEQ ID NO. 3 (scrA). In Abb. 1 und 2 sind die Plasmidkarten von pKAT 101 und pKAT 103 mit der Angabe der sequenzierten Teilabschnitte dargestellt. Abbildung 3a zeigt die Homologie dieser Abschnitte zu K.pneumoniae.

### BEISPIEL 2

### Isolierung und Charakterisierung von scr-Genen für Enzyme des Saccharose-Stoffwechsels aus Enterobacter spec. SZ 62

Es wurde eine Genbank aus Enterobacter spec. SZ 62 Gesamt-DNA in E.coli auf gleiche Weise wie in Beispiel 1 beschrieben hergestellt. Diese Genbank wurde auf Rotfärbung der Kanamycin-resistenten (25 µg/ml) Zellen auf McConkey-Saccharose-Medium gescreent. Unter den gesuchten, rot gefärbten Kolonien werden solche ausgewählt, deren Rohextrakte zugesetzte Saccharose in Glucose und Fructose umsetzen. Die aus diesen gewünschten Klonen isolierten Plasmide verleihen E.coli Wildtyp-Stämmen die Fähigkeit zum Wachstum auf Minimalsalzmedium mit Saccharose als einziger C-Quelle. Auf diese Weise wurde das Plasmid pSST3001 erhalten.

Teilfragmente aus der Insertion von pSST 3001 wurden in M13-oder pUC-Vektoren subkloniert und sequenziert. Ein Vergleich der gewonnenen Sequenzdaten mit denen in GenBank zeigt auf, daß die ermittelten Teilsequenzen hohe Homologie zu Bereichen des scr-Operons von Klebsiella pneumoniae (Jahreis und Lengeler, Supra) aufweisen. Die Teilbereiche sind auf Ebene der translatierten Sequenz zu 93-99% homolog zu Teilen der Gene für scrB, scrA, scrY und scrK des beschriebenen Organismus. Die entsprechenden Sequenzabschnitte tragen die Bezeichnung SEQ ID NO. 4 (scrR), SEQ ID NO. 5 (scrB), SEQ ID NO. 6 (scrA), SEQ ID NO. 7 (scrA), SEQ ID NO. 8 (scrY) und SEQ ID NO. 9 (scrK). In Abbildung 3b sind Sequenzen den homologen Fragmenten aus K.pneumoniae zugeordnet. Erkennbar ist, daß sich die bekannten Bereiche aus Beispiel 1 und Beispiel 2 gleichen Regionen des K.pneumoniae scr-Operons zuordnen lassen. Eine Plasmidkarte von pSST3001 mit der Angabe der sequenzierten Regionen ist in Abbildung 4 dargestellt.

### BEISPIEL 3

### Isolierung und Charakterisierung von weiteren Genen für Enzyme des Saccharose-Stoffwechsels aus P.rubrum

Aus der in Beispiel 1 angelegten P.rubrum Genbank wurde auch das Plasmid pKAT 102 erhalten. Die genomischen Anteile dieses Plasmids pKAT 102 sind nicht identisch mit denen der Plasmide pKAT 101 und pKAT 103. Die Abschnitte des Plasmids pKAT 102, die die gesuchten Gene enthalten, wurden durch Kartierung mit Restriktionsendonukleasen und Deletion von Teilfragmenten bestimmt. Plasmid-Mutanten, die den Zellen keine Saccharose-Verstoffwechselung mehr verleihen, enthalten diese über Restriktionsverdau nachweisbaren Fragmente nicht mehr.

Teilfragmente aus der Insertion von pKAT 102 wurden in M13-oder pUC-Vektoren subkloniert und sequenziert. Die entsprechenden Sequenzabschnitte tragen die Bezeichnungen SEQ ID NO. 10 (cscR), SEQ ID NO. 11 (cscA), SEQ ID NO. 12 (rafB), SEQ ID NO. 13 (gno) und SEQ ID NO. 14 (hisD). In Abbildung 5 ist die Plasmidkarte von pKAT 102 dargestellt.

Diese Sequenzabschnitte sind homolog zu Genen aus dem csc-Operon von E.coli (vgl. z.B. J. Bockmann et al., Mol. Gen. Genet. 235 (1992), 22-32) bzw. zu Genen aus Gluconobacter oder Salmonella typhimurium (vgl. Abb. 6). cscA stellt dabei ein Invertasegen und cscR das Repressorgen dar.

Es gibt Hinweise, daß csc-Gene auch in Enterobacter spec. vorhanden sind und auf analogem Weg inaktiviert werden können.

### BEISPIEL 4

### Deletion der scr-Gene für Enzyme des Saccharose-Stoffwechsels in P.rubrum

Die vorgenommene Eingrenzung der scr-Gene für die Enzyme des Saccharose-Stoffwechsels auf P.rubrum, die auf den Plasmiden pKAT 101 bzw. pKAT 103 enthalten sind, erlaubte die Festlegung der Bereiche auf den Plasmiden, die diese Gene im Genom flankieren. Relativ zur Ableserichtung der Gene (aus dem Vergleich zu K.pneumoniae) wurden ein 5'-flankierendes Fragment über KpnI-BglII aus pKAT 101 sowie ein 3'-flankierendes Fragment über BglII-SacI aus pKAT 103 jeweils isoliert und über die BglII-Schnittstelle im Vektor pUC18 verbunden. Das so entstandene Plasmid pKAT 144 enthält somit die 5'- und 3'-flankierenden Sequenzen ohne die Anteile, die für die Enzyme des Saccharose-Stoffwechsels kodieren. Entsprechend sind E.coli Kolonien, die pKAT 144 (Ampicillinresistent 50-100 µg/ml) tragen, auf McConkey-Saccharose-Medium nicht rot gefärbt. Diese Zellen sind auch auf Minimalsalzmedium mit Saccharose als einziger C-Quelle nicht wachstumsfähig. Sie enthalten also eine Deletion für die Gene, die Enzyme des Saccharose-Stoffwechsels kodieren, verglichen zu Zellen mit pKAT 101 oder pKAT 103.

Zusätzlich wurde in die singuläre BglII-Schnittstelle von pKAT144 eine Genkassette, kodierend für die Chloramphenicol Acetyltransferase (cat) eingefügt. Diese Genkassette stammt aus dem kommerziell von Boehringer Mannheim erhältlichen Plasmid pBR 328 (Soberon et al., Gene 9 (1980), 287). Aus diesem Plasmid wurde durch Spaltung mit AatII und SauI eine 1,5 kb Fragment gewonnen, das nach Behandlung der Enden mit Klenow-Enzym für die Insertion in Vektorplasmide verfügbar ist. So wurde das Plasmid pKAT 151.1 erhalten, das der Zelle zusätzlich die Resistenz gegenüber Chloramphenicol (Cm^{R}, 25-50 µg/ml) verleiht.

Fügt man dieses 5'-3'-Fragment aus pKAT 144 oder pKAT 151.1 in einen pUT-Vektor ein (Herrero, M. de Lorenzo, V., Timmis, K.N., J.Bacteriology **172:** 6557-6567, 1990), so erhält man pUT-Plasmid-Derivate, pKAT 150.1 bzw. pKAT 154.1 (Cm^{R}), die nur noch in geeigneten E.coli Wirtszellen, die das Pir-Protein zur Verfügung stellen, etabliert und repliziert werden können. Geeignet sind dafür die E.coli Stämme CC118λpir⁺ (Herrero, M., de Lorenzo, V., Timmis, K.N.J., Bacteriology **172:** (11) 6557-6567, 1990) oder S17-1λpir⁺ (de Lorenzo, V., Timmis, K.N., in Methods in Enzymology **235:** 386-405, 1994). Letzterer Stamm ist in der Lage, die pUT-Plasmid-Derivate zu mobilisieren. Kreuzt man Empfängerbakterien mit diesem Donorstamm, wird das entsprechende pUT-Plasmid-Derivat übertragen. Wenn die Empfängerbakterien das Pir-Protein nicht produzieren, ist das übertragene Plasmid nicht replizierfähig und geht verloren. Besteht aber eine Homologie zwischen den Sequenzen auf dem pUT-Plasmid-Derivat und der chromosomalen DNA des Empfängerbakteriums, so besteht die Möglichkeit der rekombinativen Integration des pUT-Plasmid-Derivates in die chromosomale DNA des Empfängerbakteriums. Dieses Ereignis ist verbunden mit der Integration der Resistenzgene des pUT-Plasmid-Derivates in die chromosomale DNA des Empfängerbakteriums.

Die verwendeten pUT-Plasmid-Derivate tragen entweder das Gen für Resistenz gegen Ampicillin (pKAT150.1) oder das Gen für Ampicillin-Resistenz und zusätzlich das Gen für Resistenz gegen Chloramphenicol (pKAT 154.1). Als Empfänger verwendet werden vorzugsweise rpoB Mutanten des P.rubrum Stammes (iiG Pr2), die resistent sind gegen Rifampicin (50-100 µg/ml). Dadurch ist eine selektive Ausschaltung der E.coli-Donorbakterien, S17-1λpir⁺, möglich. Die gewünschten Exkonjuganten werden als resistent gegen Ampicillin (100 µg/ml) und Rifampicin (50-100 µg/ml) auf Agarplatten mit Nährboullion nach Bebrütung bei 30°C für 18-36 Stunden gewonnen und auf Einzelkolonien gereinigt. Eine so gewonnene Einzelkolonie wird in Nährboullion ohne Antibiotikazusatz bei 30°C angeimpft und über Nacht kultiviert. Die gewonnene Kultur wird in einer geeigneten Verdünnungsreihe auf McConkey- Saccharose-Medium plattiert. Die nach Bebrütung bei 30°C über Nacht entstehenden Einzelkolonien sind zu 20-60% nicht mehr rot gefärbt, im Gegensatz zu den verwendeten Kontrollbakterien von P.rubrum, die nicht mit den E.coli Zellen gekreuzt worden waren. Die Zellen, die nicht rot gefärbt sind, sind nicht mehr resistent gegen Ampicillin. Diese Zellen sind auch auf Minimalsalzmedium mit Saccharose als einziger C-Quelle nicht wachstumsfähig. Sie sind aber immer noch resistent gegen Rifampicin, und dementsprechend Saccharose-Stoffwechsel-Defekt-Mutanten von P.rubrum (Stamm iiG Pr 20191). Bei Verwendung des pUT-Plasmid-Derivats pKAT 154.1, in das zusätzlich das Cm^{R}-Gen eingefügt wurde, ist auf jeder Stufe der Selektion auch ein Zusatz von Chloramphenicol (25-50 µg/ml) möglich und zweckmäßig (Stamm iiG Pr 20710).

Die auf diese Weise erhaltenen P.rubrum scr-Mutanten, z.B. iiG Pr 20191 und iiG Pr 20710, weisen die überraschende Eigenschaft auf, daß sie das Saccharose-Mutase-Enzym nunmehr konstitutiv, also ohne Zusatz von Saccharose zum Medium, produzieren, 0,5-1,5 U/mg. Im Vergleich dazu produziert der Wildtyp P.rubrum, ohne Saccharose im Medium nur ≤ 0,1 U/mg.

In den so erhaltenen Mutanten kann nach Anzucht mit Saccharose immer noch eine Invertase-Aktivität nachgewiesen werden. Es ist also neben dem charakterisierten scr-Operon mindestens noch ein weiteres Gen oder eine Gengruppe für Enzyme des Saccharosestoffwechsel in P.rubrum vorhanden, was durch die Isolierung von pKAT 102, Beispiel 3, belegt ist. Jedoch sind diese Gene (kloniert auf pKAT 102) alleine nicht ausreichend, um den beschriebenen P.rubrum scr-Mutanten ein Wachstum auf Saccharose als alleiniger C-Quelle zu ermöglichen.

Da die Mutase-Aktivität in den Mutanten nunmehr konstituiv gebildet wird, ist eine Anzucht ohne Zusatz von Saccharose im Medium möglich. Vorteilhaft ist dabei, daß diese Mutanten neben der Mutase keine Enzymaktivität besitzen, die Saccharose umsetzt. Dies führt zum Erhalt von Zellen, die zur Herstellung von Palatinose besser geeignet sind.

### BEISPIEL 5

### Deletion der scr-Gene für Enzyme des Saccharose-Stoffwechsels in Enterobacter spec. SZ 62

Die vorgenommene Eingrenzung der Gene für die Enzyme des Saccharose-Stoffwechsels auf Enterobacter spec. SZ 62, die auf dem Plasmid pSST 3001 enthalten sind, erlaubte die Festlegung der Bereiche auf dem Plasmid, die diese Gene im Genom flankieren. Die Entfernung von zwei 2,0 und 2,6 kbp großen BamHI Fragmenten durch Verdau von pSST 3001 mit diesem Enzym und Religierung erbrachte das Plasmid pIV1. Dieses Plasmid enthält somit die 5'- und 3'-flankierenden Sequenzen ohne die Anteile, die für die Enzyme des Saccharose-Stoffwechsels kodieren. Entsprechend sind E.coli Kolonien, die pIV1 (Kanamycin-resistent, 25-50 µg/ml) tragen, nicht rot auf McConkey-Saccharose. Diese Zellen sind auch auf Minimalsalzmedium mit Saccharose als einziger C-Quelle nicht wachstumsfähig. Sie enthalten also eine Deletion im Bereich der Gene, die für Enzyme des Saccharose-Stoffwechsels kodieren, verglichen zu Zellen mit pSST 3001. Zusätzlich kann in die singuläre BamHI-Schnittstelle von pIV1 eine Genkassette kodierend für die Chloramphenicolacetyltransferase (cat) eingefügt werden. So entsteht das Plasmid pIV3, das der Zelle zusätzlich die Resistenz gegenüber Chloramphenicol (Cm^{R}, 25-50 µg/ml) verleiht.

Fügt man das 5'-3'Fragment aus pIV3 entsprechend Beispiel 4 in einen pUT-Vektor ein, so erhält man das pUT-Plasmid-Derivat pIV4(Cm^{R}), das nur noch in geeigneten E.coli Wirtszellen, die das Pir-Protein zur Verfügung stellen, etabliert und repliziert werden kann. Geeignet sind dafür die E.coli Stämme CC118λpir⁺ oder S17- 1λpir⁺.

Das pUT-Plasmid-Derivat pIV4 trägt das Gen für Resistenz gegen Ampicillin und zusätzlich ein Gen für Resistenz gegen Chloramphenicol. Als Empfänger verwendet werden zweckmäßigerweise gyrA Mutanten des Enterobacter spec. SZ62 Stammes (iiG Es 2), die resistent sind gegen Nalidixinsäure (Nal^{R}, 30-50 µg/ml). Dadurch ist eine selektive Ausschaltung der E.coli-Donorbakterien, S17-1λpir⁺, möglich. Die gewünschten Exkonjuganten werden als resistent gegen Ampicillin (100 µg/ml) und Nalidixinsäure (30-50 µg/ml) auf Agarplatten mit Nährboullion nach Bebrütung bei 37°C für 18-36 Stunden gewonnen und auf Einzelkolonien gereinigt. Eine so gewonnene Einzelkolonie wird in Nährboullion ohne Antibiotikazusatz bei 37°C angeimpft und über Nacht kultiviert. Die gewonnene Kultur wird in einer geeigneten Verdünnungsreihe auf McConkey-Saccharose-Medium plattiert. Die nach Bebrütung bei 37°C über Nacht entstehenden Einzelkolonien sind zu 20-60% nicht mehr rot gefärbt, im Gegensatz zu den verwendeten Kontrollbakterien von Enterobacter spec. SZ62, die nicht mit den E.coli Zellen gekreuzt worden waren. Die nicht rot gefärbten Zellen sind nicht mehr resistent gegen Ampicillin. Diese Zellen sind auch auf Minimalsalzmedium mit Saccharose als einziger C-Quelle nicht wachstumsfähig. Sie sind aber immer noch resistent gegen Nalidixinsäure, und dementsprechend Saccharose-Stoffwechsel-Defekt-Mutanten von Enterobacter spec. SZ 62. Bei Verwendung des pUT-Plasmid-Derivats pIV4, in das zusätzlich das Cm^{R}-Gen eingefügt wurde, ist auf jeder Stufe der Selektion auch ein Zusatz von Chloramphenicol (25-50 µg/ml) möglich und zweckmäßig (Stamm iiG Es 2111, DSM 10025).

Die erhaltenen Enterobacter spec. SZ62 scr-Mutanten weisen die überraschende Eigenschaft auf, daß sie das Saccharose-Mutase-Enzym nunmehr nicht unter Zusatz von Saccharose zum Medium produzieren. Hingegen führt ein Zusatz von Palatinose zum Medium, wie auch beim Wildtyp, zur Produktion des Enzyms in einer Ausbeute von 0,5-1,5 U/mg. Die Zellen, die mit Palatinose statt Saccharose im Medium angezogen wurden, zeigen beim Umsatz von Saccharose zu Palatinose eine deutlich verringerte Bildung von Monosacchariden, wie Glucose und Fructose; die Palatinose-Ausbeute der Mutante wird dabei von 59 % auf 71,9 % erhöht (siehe Tabelle 1).

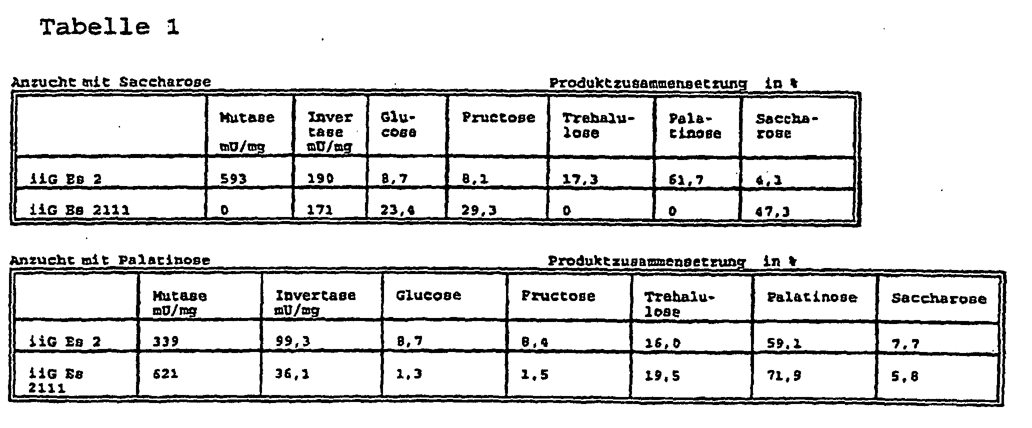

### Beispiel 6

### Deletion der csc-Gene für Enzyme des Saccharose-Stoffwechsels in P. rubrum

Gemäß der in Beispiel 4 beschriebenen Methode wurden ausgehend vom Plasmid pKAT 102 P.rubrum Saccharose-Deletionsmutanten erzeugt.

Aus pKAT 102 wurde ein 5'-flankierendes Fragment über SacI-BamHI Verdau sowie ein 3'-flankierendes Fragment über KpnI-BglII Verdau (siehe Abb. 5) jeweils isoliert und über die BamHI-BglII Schnittstellen in den pUC18 Vektor eingefügt. Somit wurde das Plasmid pSKAT9 erhalten. Alternativ wurden diese genannten Fragmente über die in Beispiel 4 beschriebene cat-Kassette verknüpft und in pUC18 eingesetzt. Dies führte zum Erhalt des Plasmids pSKAT 8. Aus diesen Plasmiden, pSKAT 9 bzw. pSKAT 8, können die 5'-3' Deletionskassetten für csc-Gene aus P. rubrum entsprechend Beispiel 4 über SacI/BamHI-, respektive HindIII, Verdau gewonnen werden und in einen pUT-Vektor eingefügt werden.
Das weitere Vorgehen war wie in Beispiel 4 beschrieben.

Auch die in csc-Genen deletierten Mutanten besitzen gegenüber den Ausgangszellen verbesserte Eigenschaften dahingehend, daß sie eine erhöhte Palatinose/Trehalulose-Ausbeute zeigen und weniger unerwünschte Mono- und Oligosaccharide als Nebenprodukte erzeugen.

### BEISPIEL 7

### Herstellung einer Protaminobacter Palatinase Defektmutante

Zellen von Protaminobacter rubrum (CBS 547, 77) wurden entsprechend der Methode von Adelberg et al. (Biochem. Biophys. Research Commun. 18 (1965), 788) modifiziert nach Miller, J., Experiments in Molecular Genetics, Cold Spring Harbor Laboratory, 125-179 (1972) mit N-Methyl-N'-nitro-N-nitrosoguanidin mutagenisiert. Zur Selektion von Palatinase-Defektmutanten wurden MacConkey-Palatinose-Medium (MacConkey-Agar-Base (Difco Laboratories, Detroit, Michigan, USA), 40 g/l unter Zusatz von 20 g/l Palatinose, sterilfiltriert, 25 mg/l Kanamycin) und Minimalsalzmedien (10,5 g K₂HPO₄, 4,5 g KH₂PO₄, 1 g (NH₄)₂SO₄, 0,5 g Natriumcitrat 2 H₂O, 0,1 g MgSO₄·7H₂O, 1 mg Thiamin, 2 g Palatinose oder Glucose, 25 mg Kanamycin und 15 g Agar pro Liter, pH 7,2) verwendet. Mutanten von P. rubrum, die auf Mc-Conkey-Palatinose-Medium weiß sind oder auf Minimalsalzmedium mit Glucose im Gegensatz zum gleichen Medium mit Palatinose wachsen, werden als Palatinase-Defekt-Mutanten identifiziert. In Zellextrakten aus den Mutanten kann die Enzymaktivität, Palatinose in Glucose und Fructose zu spalten (Palatinase-Aktivität), im Gegensatz zum Wildtyp nicht nachgewiesen werden. Züchtet man diese Zellen in Minimalsalzmedium mit 0,2 % Saccharose als einziger C-Quelle an, so ist im Gegensatz zu den Wildtypzellen, bei denen Palatinose nur transient in der Zeit von 4 bis 11 Stunden nach Kulturbeginn nachgewiesen werden kann, eine dauerhafte Akkumulation der Palatinose (Isomaltulose) nachweisbar. Übernachtkulturen in gleichem Medium enthalten im Falle der Wildtypzellen keine Palatinose, im Falle der auf diese Weise hergestellten Mutante SZZ 13 (DSM 9121) jedoch > 0,08 % Palatinose.

Gemäß den in den Beispielen 4 und 6 beschriebenen Methoden wurden ausgehend von der Palatinose-Deletionsmutante SZZ 13 und den Plasmiden pKAT 101, pKAT 103 bzw. pKAT 102 P.rubrum Saccharose-Deletionsmutanten erzeugt.

Auch diese Deletionsmutanten, die neben einem Defekt im Saccharose-Stoffwechsel einen zusätzlichen Defekt im Palatinose-Stoffwechsel aufweisen, zeigen gegenüber den Ausgangszellen (P. rubrum Wildtyp und Mutante SZZ 13) verbesserte Eigenschaften bei der Palatinose/Trehalulose-Produktion.

### BEISPIEL 8

### Immobilisierung von Mikroorganismen-Zellen

Von einer Abimpfung des entsprechenden Stammes werden Zellen mit 10 ml eines sterilen Nährsubstrats, bestehend aus 8 kg Dicksaft aus einer Zuckerfabrik (Trockensubstanzgehalt = 65 %), 2 kg Maisquellwasser, 0,1 kg (NH₄)₂HPO₄ und 89,9 kg destilliertes Wasser, pH 7,2, abgeschwemmt. Diese Suspension dient als Impfgut für die Schüttelmaschinen-Vorkultur in 1 l-Kolben mit 200 ml Nährlösung der obigen Zusammensetzung. Nach einer 30-stündigen Inkubationszeit bei 29°C werden mit 10 Kolben (Gesamtinhalt 2 l) 18 l Nährlösung der obigen Zusammensetzung in einem 30 l Kleinfermenter beimpft und bei 29°C unter Zufuhr von 20 l Luft pro Minute und einer Rührgeschwindigkeit von 350 Upm fermentiert.

Nach Erreichen von Keimzahlen über 5 x 10⁹ Keimen pro ml wird die Fermentation abgestellt und die Zellen durch Zentrifugation aus der Fermenterlösung abgeerntet. Die Zellen werden dann in eine 2 %ige Natriumalginatlösung suspendiert und durch Hineintropfen der Suspension in eine 2 %ige Calciumchloridlösung immobilisiert. Die entstandenen Immobilisatkugeln werden mit Wasser gewaschen und sind bei +4°C mehrere Wochen lagerfähig.

Zellen von Sacccharose-Stoffwechselmutanten, die gemäß den Beispielen 4 bis 6 hergestellt wurden, zeigen bessere katalytische Eigenschaften bezüglich ihrer Produktzusammensetzung als vergleichbare Zellen aus den bekannten Mikroorganismen Protaminobacter rubrum (CBS 547,77) Erwinia rhapontici (NCPPB 1578) und Enterobacter Species.

## Patentansprüche

1. Zelle, die mindestens eine für ein Protein mit einer Saccharose-Isomerase-Aktivität codierende DNA-Sequenz enthält und einen reduzierten Saccharose-Stoffwechsel aufweist, wobei die Reduzierung des Saccharose-Stoffwechsels durch teilweise oder vollständige Hemmung der Expression von mindestens einem Gen erfolgt, das für ein Saccharose-Stoffwechsel-Enzym codiert.

2. Zelle nach Anspruch 1,
**dadurch gekennzeichnet,**
**dass** das mindestens eine teilweise oder vollständig in der Expression gehemmte Gen eine DNA-Sequenz, die auf den Plasmiden pKAT 101 (DSM 10030) und pKAT 103 (DSM 10032)enthalten ist, oder eine dazu homologe DNA-Sequenz aufweist.

3. Zelle nach Anspruch 2,
**dadurch gekennzeichnet,**
**dass** die DNA-Sequenzen ausgewählt sind aus den in SEQ ID NO. 1-3 gezeigten Nucleotidsequenzen oder damit hybridisierenden Nucleotidsequenzen.

4. Zelle nach Anspruch 1,
**dadurch gekennzeichnet,**
**dass** das mindestens eine teilweise oder vollständig in der Expression gehemmte Gen eine DNA-Sequenz, die auf dem Plasmid pSST 3001 (DSM 10033) enthalten ist, oder eine dazu homologe DNA-Sequenz aufweist.

5. Zelle nach Anspruch 4,
**dadurch gekennzeichnet,**
**dass** die DNA-Sequenzen ausgewählt sind aus den in SEQ ID NO. 4-9 gezeigten Nucleotidsequenzen oder damit hybridisierenden Nucleotidsequenzen.

6. Zelle nach Anspruch 1,
**dadurch gekennzeichnet,**
**dass** das mindestens eine teilweise oder vollständig in der Expression gehemmte Gen eine DNA-Sequenz, die auf dem Plasmid pKAT 102 (DSM 10033) enthalten ist, oder eine dazu homologe DNA-Sequenz aufweist.

7. Zelle nach Anspruch 6,
**dadurch gekennzeichnet,**
**dass** die DNA-Sequenzen ausgewählt sind aus den in SEQ ID NO. 10-14 gezeigten Nucleotidsequenzen oder damit hybridisierenden Nucleotidsequenzen.

8. Zelle nach einem der Ansprüche 1-7,
**dadurch gekennzeichnet,**
**dass** sie mit einem Saccharose-Isomarasegen transformiert ist.

9. Zelle nach einem der Ansprüche 1-8,
**dadurch gekennzeichnet,**
**dass** sie eine prokaryontische Zelle ist.

10. Zelle nach Anspruch 9,
**dadurch gekennzeichnet,**
dass'sie eine gram-negative prokaryontische Zelle ist.

11. Zelle nach Anspruch 9,
**dadurch gekennzeichnet,**
**dass** sie eine Enterobakterienzelle ist.

12. Zelle nach Anspruch 9,
**dadurch gekennzeichnet,**
**dass** sie eine Escherichia coli-, Protaminobacter rubrum-, Erwinia rhapontici-, Pseudomonas mesoacidophila- oder Enterobacter spec. Zelle ist.

13. Zelle nach einem der Ansprüche 1-12,
**dadurch gekennzeichnet,**
**dass** sie das Protein mit einer Saccharose-Isomerase-Aktivität konstitutiv produziert.

14. Zelle nach Anspruch 13,
**dadurch gekennzeichnet,**
**dass** der Repressor des scr-Operons teilweise oder vollständig deletiert ist.

15. Enterobacter spec. SZ 62 Mutante
iiG Es 2111 (DSM 10025).

16. Zelle nach einem der Ansprüche 1-15,
**dadurch gekennzeichnet,**
**dass** sie weiterhin einen reduzierten Palatinose- oder/und Trehalulose-Stoffwechsel aufweist.

17. Zelle nach Anspruch 16,
**dadurch gekennzeichnet,**
**dass** die Reduzierung des Palatinose- oder/ und Trehalulose-Stoffwechsels durch teilweise oder vollständige Hemmung von Palatinase oder/und Trehalulasegenen erfolgt.

18. Verfahren zur Herstellung von Zellen, die mindestens eine für ein Protein mit einer Saccharose-Isomerase-Aktivität codierende DNA-Sequenz enthalten und einen reduzierten Saccharose-Stoffwechsel aufweisen,
**dadurch gekennzeichnet,**
**dass** man durch Mutagenese der Zelle mindestens ein Gen inaktiviert, das für ein Saccharose-Stoffwechselenzym codiert.

19. Verfahren nach Anspruch 18,
**dadurch gekennzeichnet,**
**dass** die Mutagenese durch Einführung eines zur homologen chromosomalen Rekombination geeigneten Vektors, der eine mutierte, für den Saccharose-Stoffwechsel essentielle DNA-Sequenz trägt, und Selektion von Zellen, in denen eine derartige Rekombination stattgefunden hat, erfolgt.

20. Verfahren zur Herstellung von nicht-kariogenen Zuckern, insbesondere Trehalulose oder/und Palatinose,
**dadurch gekennzeichnet,**
**dass** man zur Produktion der Zucker eine Zelle nach einem der Ansprüche 1-17 oder/und einen Extrakt aus einer derartigen Zelle verwendet.

21. Verfahren nach Anspruch 20,
**dadurch gekennzeichnet,**
**dass** man die Zelle oder den Extrakt in immobilisierter Form verwendet.

22. Verwendung von Zellen nach einem der Ansprüche 1-17 zur Herstellung von nicht-kariogenen Zuckern, insbesondere Palatinose oder/und Trehalulose.

23. Verwendung eines Mutagenese-Vektors, der eine Nucleinsäure, die für ein Enzym des Saccharose-Stoffwechsels codiert, oder ein Teilfragment davon in mutierter Form enthält, zur Herstellungvon Zellen nach einem der Ansprüche 1 bis 17.

24. Verwendung nach Anspruch 23,
**dadurch gekennzeichnet,**
**dass** die Nucleinsäure ausgewählt wird aus einem oder mehreren Genen von bakteriellen scr- oder/und csc-Operons oder Fragmenten davon.

25. Verwendung nach Anspruch 23 oder 24,
**dadurch gekennzeichnet,**
**dass** die Nucleinsäure umfasst:
(a) eine oder mehrere der in SEQ ID NO. 1-14 dargestellten Nucleotidsequenzen
(b) eine mit den Sequenzen aus (a) hybridisierende Sequenz oder/und
c) ein Fragment einer der Sequenzen aus (a) oder/und (b).

## Claims

1. A cell which comprises at least one DNA sequence coding for a protein with a sucrose isomerase activity and displays a reduced sucrose metabolism, wherein the reduction in sucrose metabolism takes place by partial or complete inhibition of the expression of at least one gene which codes for a sucrose metabolising enzyme.

2. A cell according to claim 1,
**characterised in that**
the at least one gene, the expression of which is partially or completely inhibited, comprises a DNA sequence which is contained on plasmids pKAT 101 (DSM 10030) and pKAT 103 (DSM 10032) or a DNA sequence homologous therewith.

3. A cell according to claim 2,
**characterised in that**
the DNA sequences are selected from the nucleotide sequences shown in SEQ ID NO. 1-3 or nucleotide sequences hybridising therewith.

4. A cell according to claim 1,
**characterised in that**
the at least one gene, the expression of which is partially or completely inhibited, comprises a DNA sequence which is contained on plasmid pSST 3001 (DSM 10033) or a DNA sequence homologous therewith.

5. A cell according to claim 4,
**characterised in that**
the DNA sequences are selected from the nucleotide sequences shown in SEQ ID NO. 4-9 or nucleotide sequences hybridising therewith.

6. A cell according to claim 1,
**characterised in that**
the at least one gene, the expression of which is partially or completely inhibited, comprises a DNA sequence which is contained on plasmid pKAT 102 (DSM 10033) or a DNA sequence homologous therewith.

7. A cell according to claim 6,
**characterised in that**
the DNA sequences are selected from the nucleotide sequences shown in SEQ ID NO. 10-14 or nucleotide sequences hybridising therewith.

8. A cell according to any of claims 1-7,
**characterised in that**
it is transformed with a sucrose isomerase gene.

9. A cell according to any of claims 1-8,
**characterised in that**
it is a prokaryotic cell.

10. A cell according to claim 9,
**characterised in that**
it is a Gram-negative prokaryotic cell.

11. A cell according to claim 9,
**characterised in that**
it is an enterobacterial cell.

12. A cell according to claim 9,
**characterised in that** it is an Escherichia coli, Protaminobacter rubrum, Erwinia rhapontici, Pseudomonas mesoacidophila or Enterobacter spec. cell.

13. A cell according to any of claims 1-12,
**characterised in that**
it constitutively produces the protein with a sucrose isomerase activity.

14. A cell according to claim 13,
**characterised in that**
the repressor of the scr operon is partially or completely deleted.

15. Enterobacter spec. SZ 62 mutant iiG Es 2111 (DSM 10025).

16. A cell according to any of claims 1-15,
**characterised in that**
it furthermore displays a reduced palatinose or/and trehalulose metabolism.

17. A cell according to claim 16,
**characterised in that**
the reduction in palatinose or/and trehalulose metabolism takes place by partial or complete inhibition of palatinase or/and trehalulase genes.

18. A process for producing cells which comprise at least one DNA sequence coding for a protein with a sucrose isomerase activity and display a reduced sucrose metabolism,
**characterised in that**
at least one gene which codes for a sucrose metabolising enzyme is inactivated by mutagenesis of the cell.

19. A process according to claim 18,
**characterised in that**
the mutagenesis takes place by introducing a vector which is suitable for homologous chromosomal recombination and which bears a mutated DNA sequence which is essential for sucrose metabolism, and selecting cells in which such a recombination has taken place.

20. A process for the production of noncariogenic sugars, in particular trehalulose or/and palatinose,
**characterised in that**
a cell according to any of claims 1-17 or/and an extract from such a cell is used to produce the sugars.

21. A process according to claim 20,
**characterised in that**
the cell or the extract is used in immobilised form.

22. Use of cells according to any of claims 1-17 for producing noncariogenic sugars, in particular palatinose or/and trehalulose.

23. Use of a mutagenesis vector which contains a nucleic acid, which codes for an enzyme of sucrose metabolism, or a partial fragment thereof in mutated form for producing cells according to any one of claims 1 to 17.

24. Use according to claim 23,
**characterised in that**
the nucleic acid is selected from one or more genes of bacterial scr or/and csc operons or fragments thereof.

25. Use according to claim 23 or 24,
**characterised in that**
the nucleic acid comprises:
(a) one or more of the nucleotide sequences depicted in SEQ ID NO. 1-14
(b) a sequence hybridizing with the sequences from (a) or/and
(c) a fragment of one of the sequences from (a) or/and (b).

## Revendications

1. Cellule qui contient au moins une séquence ADN codante pour une protéine ayant une activité saccharose-isomérase et présente un métabolisme réduit du saccharose, la réduction du métabolisme du saccharose se déroulant par inhibition partielle ou complète de l'expression d'au moins un gène qui est codant pour une enzyme du métabolisme du saccharose.

2. Cellule selon la revendication 1, **caractérisée en ce qu'**au moins un gène inhibé partiellement ou complètement dans l'expression présente une séquence ADN, qui est contenue sur les plasmides pKAT 101 (DSM 10030) et pKAT 103 (DSM 10032) ou une séquence ADN analogue à celle-ci.

3. Cellule selon la revendication 2, **caractérisée en ce que** les séquences ADN sont choisies parmi les séquences de nucléotides présentées dans SEQ ID NO. 1-3 ou les séquences de nucléotides hybridisant avec celles-ci.

4. Cellule selon la revendication 1, **caractérisée en ce qu'**au moins un gène inhibé partiellement ou complètement dans l'expression, présente une séquence ADN qui est contenue sur le plasmide pSST 3001 (DSM 10033) ou une séquence ADN homologue à celle-ci.

5. Cellule selon la revendication 4, **caractérisée en ce que** les séquences ADN sont choisies parmi les séquences de nucléotides représentées dans SEQ ID NO. 4-9 ou les séquences de nucléotides hybridisant avec celles-ci.

6. Cellule selon la revendication 1, **caractérisée en ce qu'**au moins un gène partiellement ou complètement inhibé dans l'expression présente une séquence ADN, qui est contenue sur le plasmide pKAT 102 (DSM 10033) ou une séquence ADN homologue à celle-ci.

7. Cellule selon la revendication 6, **caractérisé en ce que** les séquences ADN sont choisies dans les séquences de nucléotides représentées dans SEQ ID NO. 10-14 ou dans les séquences de nucléotides hybridisant avec celles-ci.

8. Cellule selon l'une quelconque des revendications 1 à 7, **caractérisée en ce qu'**elle est transformée avec un gène de saccharose isomérase.

9. Cellule selon l'une quelconque des revendications 1 à 8, **caractérisée en ce qu'**elle est une cellule procaryote.

10. Cellule selon la revendication 9, **caractérisée en ce qu'**elle est une cellule procaryote Gram-négative.

11. Cellule selon la revendication 9, **caractérisée en ce qu'**elle est une cellule d'entérobactérie.

12. Cellule selon la revendication 9, **caractérisée en ce qu'**elle est une cellule d'*Escherichia coli, Protaminobacter rubrum, Erwinia rhapontici, Pseudomonas mesoacidophilia* ou *Enterobacter spec*.

13. Cellule selon l'une quelconque des revendications 1-12, **caractérisée en ce qu'**elle produit de façon constitutive la protéine ayant une activité saccharose isomérase.

14. Cellule selon la revendication 13, **caractérisée en ce que** le répresseur de l'opéron scr est partiellement ou entièrement délété.

15. *Enterobacter sp SZ 62 mutantes iiG Es 2111* (DSM 10025).

16. Cellule selon l'une quelconque des revendications 1 à 15, **caractérisée en ce qu'**elle présente par ailleurs un métabolisme réduit du palatinose et/ou du tréhalulose.

17. Cellule selon la revendication 16, **caractérisé en ce que** la réduction du métabolisme du palatinose et/ou du tréhalulose intervient par l'inhibition partielle ou complète de la palatinase et/ou des gènes de tréhalulase.

18. Procédé de fabrication de cellules qui contiennent au moins une séquence ADN codante pour une protéine ayant une activité saccharose-isomérase et présentent un métabolisme réduit du saccharose, **caractérisé en ce qu'**on inactive, par mutagenèse de la cellule, au moins un gène qui est codant pour une enzyme métabolique du saccharose.

19. Procédé selon la revendication 18, **caractérisé en ce que** la mutagenèse intervient par introduction d'un vecteur convenant pour la recombinaison chromosomale homologue qui porte une séquence ADN mutée essentielle pour le métabolisme du saccharose et par sélection de cellules dans lesquelles une recombinaison de ce type a eu lieu.

20. Procédé de fabrication de sucres non cariogènes, en particulier de tréhalulose et/ou de palatinose, **caractérisé en ce qu'**on utilise une cellule selon l'une quelconque des revendications 1 à 17 et/ou un extrait d'une telle cellule pour produire les sucres.

21. Procédé selon la revendication 20, **caractérisé en ce qu'**on utilise la cellule ou l'extrait sous forme immobilisée.

22. Utilisation de cellules selon l'une quelconque des revendications 1 à 17 pour fabriquer des sucres non cariogènes, en particulier le palatinose et/ou le tréhalulose.

23. Utilisation d'un vecteur de mutagenèse qui contient un acide nucléique, qui est codant pour une enzyme du métabolisme du saccharose, ou un fragment partiel de celui-ci sous forme mutée, pour fabriquer des cellules selon l'une quelconque des revendications 1 à 17.

24. Utilisation selon la revendication 23, **caractérisée en ce que** l'acide nucléique est choisi parmi un ou plusieurs gènes d'opérons bactériens scr et/ou csc ou des fragments de ceux-ci.

25. Utilisation selon la revendication 23 ou 24 **caractérisée en ce que** l'acide nucléique renferme :
(a) un ou plusieurs séquences représentées dans SEQ ID NO. 1-14
(b) une séquence hybridisant avec les séquences de (a) et/ou
(c) un fragment d'une des séquences de (a) et/ou de (b).


### SEQUENZPROTOKOLL

(1) ALLGEMEINE ANGABEN:
   (i) ANMELDER:
      (A) NAME: Suedzucker Aktiengesellschaft Mannheim/Ochsenfurt
      (B) STRASSE: Maximilianstr. 10
      (C) ORT: Mannheim
      (E) LAND: Deutschland
      (F) POSTLEITZAHL: 68165
   (ii) BEZEICHNUNG DER ERFINDUNG: Saccharose-Stoffwechsel-Mutanten
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 863 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 1:
   ATGAACGTTA TCTCAACGTT TAVCCAGRCG GGCTATCTGA CCGGCACGCT GGATTATCGA    60
   CAGGCGGCCT TTGAACATGG CGAATTCCAC GAGCTGGACG CCGGGTTCGA ATTCTATGCG   120
   CCGCAAACCA CGCTGCCGAA GACGGCCGCC GCCTGCTGGT CGGCTGGATG GGCGTACCGG   180
   AGCAGGACGA AGTCTGTCAC CCGACGTTGC AATACGGCTG GATCCATACC ATGACCTGCC   240
   CGCGCGAACT TTCTTTGCAG AACGGCAAGC TTTACCAGCA GCCGGCGCGC GAGCTGCAAC   300
   GCGTGCGCGG CGAGGGCAGC CAATGGCAGG GAATGGCGGG TAACGCCCCT GTCTGGCCGG   360
   TCGACAGTGC CGAAGTGTTG TTGACGCCCA ACGGCGCATT CAGCGCCGAC TTTGGCGATG   420
   CGATGACGCT GAGCTGGGAC GGCGCCTTGC TGCGTCTGAC GCGCAACAAC CTGCGCAACG   480
   GGCAGCCTGA ACACCGTTAC TGGCGCGGTG AGGTCACCCA TCTGCAAATG CTGTTCGACA   540
   GCTCCAGCGT GGAGATTTTT ATCAACCACG GCGAAGGGGT CATGAGCTCA CGCTACTTCC   600
   CTGGCCCGCA ACCGTTGCTG CGTTTGAGCG GTGAGACGCA GCTTGCACTG GAGCACTGGC   660
   CGCTGACGCC ATGCATGCTA GAATGACGCA TCTCCCGTTG AACTCAGATG CCGCCAGGTG   720
   AAAAAAAACA AACGATAACC ATCAGTGATA TTGCCGCGCT GGCCGGCGTG TCGAAATCGA   780
   CCGCCAGCCT GGTGCTTAAC GGACGCAGCA AAGAATATCG GGTGTCCGAC GACACGTGAC   840
   GTATCTGCGC TGGCGGCGAG CAC                                            863
(2) ANGABEN ZU SEQ ID NO: 2:
   (i) SEQUENZKENNZEICHEN:
      (A) LÄNGE: 329 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 2:
   TCTATCAGTG GAACCCGCTG GGCTGCGATC ACCGCAACAA ATGCTGGGGC CATTGGCAAT     60
   CGACGGATCT GCTCAACTGG CAGCATCAGC CGGTGGCGCT GGTGCCCGGC GCTTGTTACG    120
   ACAGSCASGG CTGTTACTCA GGCTCGGCGG TGGTGGCAGA CGGCAAAATC ATGCTGGCGT    180
   ATACCGGCAA CGTAAAATAC CCCGACGGTT CTCGCACAGC GTACCAGTGC CTGGCGYYGG    240
   AAAATGCGCA GGGCGGGTAT GACAAGCTCG GCCCGGTGCT GCCGCTGCCG GAAGCTACAG    300
   CGGCCACGTG CGCGATCCNA AGGTTTGGC                                      329
(2) ANGABEN ZU SEQ ID NO: 3:
   (i) SEQUENZKENNZEICHEN:
      (A) LÄNGE: 465 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 3:
   GATTATTGGC CATCGACCAG ATCGGCAGCC AGGAAGTTAA CGCCGATTTC CGGGTTGCCG     60
   AGCAGCCCCC CCTCTACGGC GTGGAAGCTG TGGTGAATGC CGGTGATGAC GATGGCGGAA    120
   TACAGGCCGC CGAACAGGAA TCCGGCGAAC CAACCGGCGT GGGCAATCAG CGTGCTGAGC    180
   ACGAAGGAGA TGCCGTCACC CAGTGTGCGC CCGGCGGGGC CGATAAACAG CATGGCGACG    240
   AAGCCGGAGA TCACCACCGT CAGGAATGGC GTCAGGATCA GGTCCAGCGC GTTGGGGATC    300
   ACTCTGCGCA GCTGTTTTTC CAGCACGCTC ATAAACCACA CCGTCAGCAG CACCGGGAAC    360
   ACCGTACCCT GATAGCCGAT CATGGCGATA TCGAGTCCGA AGAAATTCAT GGTGTGGAAA    420
   CCGCCCGCGA CGCCCCAGGC GTTGGTCAGC GCCGGGTGAG TCAGG                    465
(2) ANGABEN ZU SEQ ID NO: 4:
   (i) SEQUENZKENNZEICHEN:
      (A) LÄNGE: 308 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 4:
   TTTTCTCCCA TGAGCTTGAG ACGCTCTGCC GGGAAGCTGG BGKGCAGCTG CTTATCTCCT    60
   GCACCGACGA AAACCCCGGC CAGGAGAGCG TGGTGGTCAA TAATATGATT GCCCGCCAGG   120
   TCGACGGGCT GATCGTCGCC TCGTGCATGC ACAGCGATGC CGACTACCTG AAGCTCAGCG   180
   AACAGCTGCC GGTGGTGCTT TTTGACCGCT CCCCCAGCGA CAGCGCCCTG CCGCTGGTGA   240
   TGACCGACTC GGTGACGCCA ACCGCCGAGC TGATCTCCCG CATTGCGCCT CAGCATGCGG   300
   ACGAATTC                                                            308
(2) ANGABEN ZU SEQ ID NO: 5:
   (i) SEQUENZKENNZEICHEN:
      (A) LÄNGE: 335 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 5:
   TGGCGTTAAC GGTCTGGCGA ACGCGGGCTA TATGTGGGAG TGCCCGGATC TCTTTCCGCT    60
   GGCGGATACC TACCTGCTGA TCTGCTGCCC GCAGGGGCTG GCCCGTGAAG CGCAGCGCTT   120
   TCTCAATACC TATCCTGCGG TGTGGATGGC AGGCCGCTTC GACGCCGAGC GCGGCACCTT   180
   CAACCACGGC CCGCTGCACG AGCTGGACAG CGGGTTTGAG TTCTACGCGC CGCAGACCAT   240
   GCTGGCCGAG GATGGCCGCC GTCTGCTGGT CGGCTGGATG GGCGTCCCGG ACGGGGAAGA   300
   GATGCATCAA CCCACCCGCG CACAGGGATG GATCC                              335
(2) ANGABEN ZU SEO ID NO: 6:
   (i) SEQUENZKENNZEICHEN:
      (A) LÄNGE: 357 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 6:
   GGATCCTCAC CCACCCGGCG CTGACCAACG CCTGGGGCGT CGCCGCCGGC TTCCACACCA     60
   TGAATTTCTT CGGCATCGAA GTGGCGATGA TCGGCTACCA GGGCACCGTC TTCCCGGTGC    120
   TGCTGGCGGT GTGGTTTATG AGCATGGTCG AGAAACGGCT GCGCSSCGTT ATCCCTGACG    180
   CGCTGGACCT GATCCTCACC CCGTTCCTGA CGGTGATTAT CTCCGGCTTT ATCGCCCTGC    240
   TGCTGATCGG CCCGGCCGGT CGCGCGCTCG GCGACGGTAT TTCGTTTATC CTCAGCACGC    300
   TTATCAGCCA CGCCGGCTGG CTGGCGGGCC TGCTGTTCGG CGGCCTCTAC TCGGTGA       357
(2) ANGABEN ZU SEQ ID NO: 7:
   (i) SEQUENZKENNZEICHEN:
      (A) LÄNGE: 242 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 7:
   CCGCCTGCTK TCCAACATTT TCGTACCGAT CATTCCCGCC ATCGTCSSCT CCGGCCTGCT     60
   GATGGGCCTG CTGGGGATGG TGAAAACCTA CGGCTGGGTC GACCCGAGCA ACGCTATCTA    120
   TATCATGCTG GATATGTGCA GCTCGGCAGC GTTTATCATT CTGCCGATCC TGATCGGCTT    180
   TACCGCCGCC CGCGAATTTG GCGGTAACCC TTATCTGGGC GCGACCCTTG GCGGGATCCC    240
   CG                                                                   242
(2) ANGABEN ZU SEQ ID NO: 8:
   (i) SEQUENZKENNZEICHEN:
      (A) LÄNGE: 255 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 8:
   CTGCAGGACG CCGAAACCCG CGCCAGCACT GCCGAAAGCC GTGCCGCCTC GGCGGAGCAG    60
   AAAGTTCAGC AGTTAACCCA GCAGCAGCAG CAAACCCAGG TCACCACCCA GCAGGTGGCC   120
   AAGCGCACCA CTCAGCTGGA AGAAAAAGCC GAACGGCCTG GCGGCTTTGA GTTCCACGGC   180
   TATGCCCGTT CCGGCGTGAT CATGAACGAC TCGGCCGCCA GCACCAAATC CGGCGCTTAT   240
   ATGACCCCCG CCGGG                                                    255
(2) ANGABEN ZU SEQ ID NO: 9:
   (i) SEQUENZKENNZEICHEN:
      (A) LÄNGE: 246 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Enterobacter species
   (xi) SEQUENZBESCHREIBUNG: SEQ ID NO: 9:
   HTCTAGAGGA TCCGCAGGAC CTGCGTGACT GTCTCGACCG GGCGCTGGCC CTCGCCGACG    60
   CCATAAAACT CTCGGAAGAG GAGCTGGCCT TTGTCAGCGG CAGCGACGAC ATCGTCAGCG   120
   GCATCGCCCG GCTGAACGCC CGCTTCCAGC CGACGCTGCT GCTGGTGACC CAGGGCAAAG   180
   CGGGGGTCCA GGCCGCCCTG CGCGGGCAGG TTAGCCACTT CCCCGCTCGC CCGGTGGTGG   240
   CCGTTG                                                              246
(2) ANGABEN ZU SEQ ID NO: 10:
   (i) SEQUENZKENNZEICHEN:
      (A) LÄNGE: 293 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 10:
   GGATCCTGTC GATCGAAAAG ACTGCGCGCG AACACGGCTG GAACAGCTTT GTCGTCAACC    60
   TGTTCGCCGA CGACAGCGCC GAACACACCA TAGACTTGCT GCTGCCGCAC CGGCCGGATG     120
   GGGTGATTTT CACCACCATG GGGCTGCGAC AGGTCGCCGT GCCGCCCAAG TTACTGGATA     180
   AAAAGCTGGT GCTGGCCAAC TGCGTCAGCA GGGAAATAGT GTTGCAGCTA CATTCGGATG     240
   ACGAACAGGG CCAGTACGAC GCCATACGCG CGTTGATCGC CAAAGGCTAC CGY            293
(2) ANGABEN ZU SEQ ID NO: 11:
   (i) SEQUENZKENNZEICHEN:
      (A) LÄNGE: 919 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 11:
   GCTGCGCGGC GAGACTCCCG CCCGCGCACC TCAATTTCAA TCCACGATAA AATCAAGTGA      60
   GCCAATCCAA TGAACAATGC CTTAGCTCAA GCCGACCATG CGGTCGAAAC CCTGCGCGCA     120
   CAGCGTCAAG ACGATTACTA CCCGCAATTT CATCTGGCGC CTGCCGCCGG CTGGATCAAC     180
   GATCCCAATG GGTGGGTGTA CATTAACGGC GTTTATCATG CGTTCTATCA ACACCACCCC     240
   TACGACGRAA ACTGGGGAMC GATGCACTGG GGCCACGCCA TCAGCCGGGA TCTGGCGCAC     300
   TGGCAGCACC AGCCGATAGC CCTGTGCGCC CGGCGACGAC TACGACAAAG ACGGCTGTTT     360
   CTCCGSTGCG CAGTGGACGA TAACGGCGTG CTGACGCTGA TCTACACCGG CCACGTCTGG     420
   CTGGACAAAG TGGGCGATGA CGATCAGGTA CGCGAGGTAC AGTGCCTGGC CACCAGCGAG     480
   GACGGCGTAC ATTTCGTCAA GCACGGGCCG GTGTTGGCGC CGCCGAAAGG CATTCAGCAT     540
   TTCCGCGATC CCAAGGTCTG GCGCGAGAGT GACGGCTGGT GGATGGTGGT CGGCGCCAAA     600
   CAAAACGGCC TCGGGCAGGC ACGGCTTTAT CGTTCAAAAG ATCTGCGTGA CTGGCAGTTT     660
   GACCGCGTGC TGGACGGCGC GCAGACGCTG CATCAGGGCT ATATGTGGGA GTGCCCGGAT     720
   TTCTTCCCTC TCGGCGAAAA ACAGGTGCTG CTGTTCTCGC CGCAGGGSCT GGCGGCACAG     780
   GGCTACCGCC ACCGTAACCG CTTCCAGAGC GGCTATTTGC TCGGTCACTG GCAGCCGGGC     840
   GCTGATTTCA ACGTCACTCA ACCTTTCTGC GAACTGGACG CCGGGCATGA TTTCTATGCG     900
   CCGCAAAMCT TTACMGSCG                                                  919
(2) ANGABEN ZU SEQ ID NO: 12:
   (i) SEQUENZKENNZEICHEN:
      (A) LÄNGE: 327 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 12:
   AYTCGGTATC AAACAAAGCC TGCTGCTGGC CAGCAGCGTG GATGGGCGTT TCGCATGTTC      60
   GGCTCCGGCT TCGCCAACGG CGCGCTGATG ATTTCCGCCA TGAAACTGCT GCATGCCGTG     120
   GAGCTGCCGA TTCTGCTGGT AGCCATGTTC AAATACATCA CCACCCGCTT TGACAGCCGC     180
   CTGTCCTCCA CGCTGTACCT AGTGGGCTTC CAGTTTATCA GCCAGATCGT CGCCGGCATT     240
   CTGGCGCCCA GCATGGCCAG GTCATCGGCT ACGACCGCAT CGGTTTTGCC GACACCTATT     300
   TGCTGATGGT GCGCCGTCGC CRGTACC                                         327
(2) ANGABEN ZU SEQ ID NO: 13:
   (i) SEQUENZKENNZEICHEN:
      (A) LÄNGE: 1014 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 13:
   CGTGTTGAAA CCCTGGTGGT TCCCTGAGGA GAATTTATGC AAAACACCCA TTTGCCGCCG      60
   GGCCTGGCCC CCTTTTCATT GCTCGGCAAG CGTGCACTGA TCACCCGGGC TACGCGAGGG     120
   ATTGGTCAGG CGCTGGCGAT CGGGCTGGCG CAGGCCGGTG CGCAGGTGAT CGTCGCCGGG     180
   CGACAACGCG CCGCGCTGCA AGAGGTGGTA CAACAGCTGA ATAATTGGGG AGAACACCCG     240
   GAAATGCTGC TGTTGGATGT CCAGGATCCG GCGTCGATTG AAACCGCCTT TGCCACGCTG     300
   GCGGGCAAAC CGCTGGATAT CCTGATCAAC AACGCCGGTA TCGAACGCTT GTGCCCGTCG     360
   CTGGAGGTGG ATGAAACGCT GTGGGACAGC GTCGTCGGCA CCAACCTTAA AGGCGCTTTC     420
   TTCTGCGNGC AGGCGGCGGC GCGCCTGATG GTCAAACAGG GCAGCGGCAG CATCATCAAC     480
   CTGTGCTCAT TGACCAGCGA AGTCGGCGTG CCGGGGGCTH ACCTACGGCG CGTCAAAATC     540
   CGGGCTGGCC GGCATGACGC GTGCGCTGGC CAGCGAATGG GCTACTACGG GATCCGGGTC     600
   AACGGCATCG GGCCAGGCTA TTTTCAGACC GCGATGACCG AGGTGTTTTA CCAGGACAAC     660
   GGTTGGCGCG AGTCCATGCA GGATAAAATT CCGCTAGGCC GTTTTGGCAA GCTGAGCGAC     720
   CTTACCGGTG CGGCCATTTT CCTCGCCGGC CCGGCGGCGG CCTACATCAC CGGCCAGATC     780
   CTGTATGTCG ACGGCGGCTA TCTGGCCAGC ATCTGAATAA ACGCAGGCGT GGGCGATATC     840
   CCGCCCACTC TCCAGCGCCT TGCATCCATC CAGTGATGAC GATCACAACG CAAATATGTT     900
   AACGTTAACT TTTGTGATTA ACATCGCAAT CCCTGATGTT GGTGGCTTTA TTTGGCGAAA     960
   GGTTAACGTT AACAATAAGG STGACATCAT CCATCAGGGS ATCTCACATG AACN          1014
(2) ANGABEN ZU SEQ ID NO: 14:
   (i) SEQUENZKENNZEICHEN:
      (A) LÄNGE: 335 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNLICHE HERKUNFT:
      (A) ORGANISMUS: Protaminobacter rubrum
   (xi) SEQUENZBESCHREIBUNG: SEQ ID NO: 14:
   GGATCCCACC GTCACCGCCG CTATCGCCTA TGCCGCCGGC AATATCCGCG CTTTTCACGA    60
   AGCGCAGAAA CCGGAAGAAA TGTGGTTGAA AGAGATGCAG CCCGGCGCTT TTGCCGGCGA   120
   TCGCCACCTG CCGATCGATT CCGTAGCCTG CTATGTGCCG CGCGGCAAAG GTTCGTTCCC   180
   CAGCGTGCTG CTGATGACGG CGATCCCGGC GCTGGTGGCC GGCGTACCCA GGCCGGTGGT   240
   GATTACCCCS CCCGGCCCGG ATGGCAGAGT CGATGATGCC ACGCTGGTGG VGGCGCAGTT   300
   GGTCGGCATC CGCGAAATTT ACAAGTGGCG GGGGG                              335